(19) **Europäisches Patentamt / European Patent Office / Office européen des brevets**

(11) **EP 4 495 937 A1**

(12) **EUROPEAN PATENT APPLICATION**
published in accordance with Art. 153(4) EPC

(43) Date of publication:
**22.01.2025 Bulletin 2025/04**

(21) Application number: 23770309.5

(22) Date of filing: **22.02.2023**

(51) International Patent Classification (IPC):
**G16C 20/40** (2019.01)

(52) Cooperative Patent Classification (CPC):
**G16C 20/40**

(86) International application number:
**PCT/JP2023/006506**

(87) International publication number:
**WO 2023/176350 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.03.2022 JP 2022043568**
**24.01.2023 JP 2023008912**

(71) Applicant: **Panasonic Intellectual Property Management Co., Ltd.**
**Kadoma-shi, Osaka 571-0057 (JP)**

(72) Inventors:
- **ICHIKAWA Kazuhide**
  **Kadoma-shi, Osaka 571-0057 (JP)**
- **OHUCHI Satoru**
  **Kadoma-shi, Osaka 571-0057 (JP)**
- **YOKOYAMA Tomoyasu**
  **Kadoma-shi, Osaka 571-0057 (JP)**
- **UENO Koki**
  **Kadoma-shi, Osaka 571-0057 (JP)**

(74) Representative: **Grünecker Patent- und Rechtsanwälte**
**PartG mbB**
**Leopoldstraße 4**
**80802 München (DE)**

(54) **INFORMATION PROCESSING SYSTEM, INFORMATION PROCESSING DEVICE, CALCULATION DEVICE, INFORMATION PROCESSING METHOD, AND CALCULATION METHOD**

(57) An information processing system (1000) includes: an information processing apparatus (100) that transmits information pertaining to a relational expression representing energy of a structure of an aggregate of atoms; and an optimization apparatus (200), which is an example of a computing apparatus that performs computation using the relational expression. The relational expression is acquired on the basis of (a) atomic information pertaining to the states of the two or more atoms included in an aggregate and (b) position information pertaining to two or more positions where the states of atoms in the aggregate may be located. The relational expression includes two or more variables that indicate the states of atoms located at the two or more positions. The information processing apparatus (100) includes: a first communicator (102) that transmits the information pertaining to the relational expression to the optimization apparatus (200), and receives, from the optimization apparatus (200), values of the two or more variables derived by performing the above computation; and an outputter (103) that outputs structure information corresponding to the values of the two or more variables.

FIG. 2

## Description

Technical Field

The present disclosure relates to a technology for performing processing related to the structure of aggregates of atoms, such as crystals.

Background Art

NPL 1 discloses a technique for computationally finding stable crystal structures using the cluster-expansion approach and Monte Carlo simulation. PTL 1 discloses, among other things, a mixture performance optimization apparatus. In this mixture performance optimization apparatus, an energy function expression is used in computation to optimize the performance of a mixture by individually weighting physical properties of the mixture. This enables the mixture performance optimization apparatus to optimize the performance of a mixture not only as a computed result but also as real performance, even in cases where the mixture has many physical properties. PTL 2 discloses, among other things, a crystal material analysis apparatus that converts two crystal structures to graphs to compute structural similarity. PTL 3 discloses a design program for designing the composition of a stable perovskite-type crystal structure.

Citation List

Patent Literature

PTL 1: Japanese Unexamined Patent Application Publication No. 2021-127418
PTL 2: Japanese Unexamined Patent Application Publication No. 2021-028780
PTL 3: Japanese Unexamined Patent Application Publication No. 2021-033768 Non Patent Literature

NPL 1: Zijian Yang, Robyn E. Ward, Naoto Tanibata, Hayami Takeda, Masanobu Nakayama, and Toru Asaka, Journal of Physical Chemistry C, 2020, 124, 9746 "Arrangement in La1/3NbO3 Obtained by First-Principles Density Functional Theory with Cluster Expansion and Monte Carlo Simulation"

Summary of Invention

However, there is a problem in that even if the methods, apparatuses, and the like of NPL 1, PTL 1, PTL 2, and PTL 3 above are used, it is difficult to improve the efficiency of the processing for searching for the structures of aggregates of atoms.

The present disclosure addresses this problem and provides an information processing system with which improved efficiency can be attained in the processing for searching for the structures of aggregates of atoms.

To address the above problem, an information processing system according to a first aspect of the present disclosure includes: an information processing apparatus that transmits information pertaining to a relational expression representing energy of a structure of an aggregate of atoms; and a computing apparatus that receives the information pertaining to the relational expression and performs computation using the relational expression. The aggregate of atoms includes states of two or more atoms. The relational expression is acquired on the basis of (a) atomic information pertaining to the states of the two or more atoms included in the aggregate and (b) position information pertaining to two or more positions where any of the states of the two or more atoms in the aggregate may be respectively located. The first relational expression includes two or more variables that indicate the states of atoms respectively located at the two or more positions. The information processing apparatus includes: a communicator that transmits the information pertaining to the relational expression to the computing apparatus, and receives, from the computing apparatus, values of the two or more variables derived by performing computation using the relational expression; and an outputter that outputs structure information indicating a structure of the aggregate that corresponds to the values of the two or more variables.

It should be noted that general or specific embodiments may be implemented by an apparatus, a method, an integrated circuit, a computer program, a computer-readable recording medium, or any selective combination thereof. The computer-readable recording medium includes a non-transitory recording medium such as a Compact Disc - Read-Only Memory (CD-ROM).

According to the present disclosure, improved efficiency can be attained in the processing for searching for the structures of aggregates of atoms.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

Brief Description of Drawings

[Fig. 1] Fig. 1 is a diagram illustrating an example of a configuration of an information processing system according to Embodiment 1.
[Fig. 2] Fig. 2 is a block diagram illustrating an example of a functional configuration of an information processing apparatus and an optimization apparatus according to Embodiment 1.
[Fig. 3] Fig. 3 is a diagram for explaining an example of processing by the information processing apparatus according to Embodiment 1.
[Fig. 4] Fig. 4 is a diagram illustrating an example of coefficients obtained by the information processing apparatus according to Embodiment 1.
[Fig. 5] Fig. 5 is a diagram for explaining an example of processing by the optimization apparatus and the information processing apparatus according to Embodiment 1.
[Fig. 6] Fig. 6 is a flowchart illustrating an example of processing operations by an information processing system according to Embodiment 1.
[Fig. 7] Fig. 7 is a flowchart illustrating in detail an example of the processing in step S11 of Fig. 6.
[Fig. 8] Fig. 8 is a diagram illustrating a specific example of the processing in steps S111 and S113 of Fig. 7.
[Fig. 9] Fig. 9 is a diagram illustrating a specific example of the processing in steps S114 and S115 of Fig. 7.
[Fig. 10] Fig. 10 is a flowchart illustrating in detail an example of the processing in step S12 of Fig. 6.
[Fig. 11] Fig. 11 is a diagram illustrating a specific example of the processing in step S121 of Fig. 10.
[Fig. 12] Fig. 12 is a diagram illustrating a specific example of the processing in steps S122 and S123 of Fig. 10.
[Fig. 13] Fig. 13 is a diagram illustrating another specific example of the processing in steps S122 and S123 of Fig. 10.
[Fig. 14] Fig. 14 is a diagram illustrating another specific example of the processing in step S121 of Fig. 10.
[Fig. 15] Fig. 15 is a diagram illustrating another specific example of the processing in steps S122 and S123 of Fig. 10.
[Fig. 16] Fig. 16 is a block diagram illustrating an example of a functional configuration of an information processing apparatus and a sampling apparatus included in an information processing system according to Embodiment 2.
[Fig. 17] Fig. 17 is a diagram for explaining a Boltzmann distribution.
[Fig. 18] Fig. 18 is a diagram for explaining an example of processing by the sampling apparatus and the information processing apparatus according to Embodiment 2.
[Fig. 19] Fig. 19 is a flowchart illustrating an example of processing operations by an information processing system according to Embodiment 2.
[Fig. 20] Fig. 20 is a diagram illustrating an example of additional processing operations by a first controller according to Embodiment 2.
[Fig. 21] Fig. 21 is a block diagram illustrating an example of a functional configuration of an information processing apparatus and a computing apparatus included in an information processing system according to Embodiment 3.
[Fig. 22] Fig. 22 is a flowchart illustrating an example of processing operations by an information processing system according to Embodiment 3.
[Fig. 23] Fig. 23 is a flowchart illustrating an example of processing operations by an information processing system according to Modification 1 of Embodiment 3. Description of Embodiments

(Underlying Knowledge Forming Basis of the Present Disclosure)

In recent years, materials development has involved corroborating the physical properties of synthesized materials through computation, and computationally predicting the physical properties of unknown materials. In particular, an approach referred to as first-principles computation is widely used because this approach allows many physical properties of a material to be computed if the structure of the material (the arrangement of atoms that form the material) is understood. However, since the structures of materials that can actually be synthesized are energetically stable, it is necessary to know the stable crystal structure of a material to computationally predict the physical properties of the material. In most cases, the stable structure of a material can be approximated well by the structure with the lowest energy. However, in many cases, such as the case of a crystal or cluster in a multidimensional system that is an aggregate of two or more types of atoms, only the positions where any of the atoms may be located (hereafter also referred to as sites or site positions) are known. Therefore, in many cases, it is not known which atoms (or vacancies) located at which sites in the aggregate will result in a stable structure of the aggregate.

For example, oxides of transition metal elements are often used as positive electrode materials in Li-ion secondary batteries, but in some cases, performance (such as electric potential and capacity) is improved by using more than one transition metal elemental species, and the degree of improvement depends on the ratio of transition metal species. A technique for determining stable structures is very important for materials development, since it is necessary to know which transition metals located at which crystalline sites will result in stability to computationally predict the ratio at which performance is optimal. Such a technique would also be useful for negative electrode materials, solid electrolyte materials, high-temperature structural materials, biological materials, fusion reactor structural materials, electrolytic capacitor materials, catalyst materials, and so on.

A conventional technique for determining stable crystal structures involves determining an optimal arrangement of atoms with respect to energy. In this technique, the energy of the crystal structure corresponding to a particular arrangement combination is computed for each and every arrangement combination, and the arrangement combination with the lowest energy is found. Note that an arrangement combination is a combination of atoms respectively located at sites. The arrangement combination with the lowest energy is the optimal arrangement of atoms mentioned above.

NPL 1 discloses a technique that involves using a method referred to as the cluster-expansion approach to compute the energy of a crystal structure, and using a Monte Carlo method (specifically, simulated annealing) search for the arrangement combination with the lowest energy. In this technique, by using the cluster-expansion approach, the cost of computing the energy of each crystal structure can be lowered without much loss of accuracy, and by further using a Monte Carlo method, an optimal arrangement can be found without computing all arrangement combinations. However, when the number of arrangement combinations is huge, it is necessary to compute the energy of the crystal structure corresponding to each of a very large number of arrangement combinations to find a stable crystal structure, even with techniques for finding stable crystal structures using Monte Carlo methods. As a result, the technique disclosed in NPL 1 requires large amounts of computational resources to determine stable crystal structures.

Meanwhile, recent years have seen the brisk development of dedicated machines specialized in solving combinatorial optimization problems, and many application examples have been disclosed. PTL 1 discloses a technique for computing a mixing ratio so that performance is optimized for a mixture of materials. More specifically, this technique acquires physical property values of materials before the materials are mixed, converts an energy function expression for performing computation to optimize the performance of a mixture to a function in Ising form, and uses an annealing machine to output an optimal mixing ratio.

PTL 2 discloses a technique for computing the structural similarity of two crystal structures. More specifically, this technique converts two crystal structures into respective labeled graphs, converts the two graphs into a single graph referred to as a conflict graph, and computes the structural similarity by obtaining the maximum independent set in the conflict graph. Also, in PTL 2, in obtaining the maximum independent set in the conflict graph, a function in Ising form is constructed from the conflict graph, and the function in Ising form is solved using an annealing machine or the like.

PTL 3 discloses an optimization technique using a function in Ising model form for perovskite-type crystal structures. More specifically, a composition ratio of atomic species that gives a stable perovskite-type crystal structure is obtained.

However, crystal structures are not outputted by the techniques in PTL 1, PTL 2, and PTL 3. Even with the technique in PTL 3, only a composition ratio of a perovskite-type crystal structure is obtained, and information indicating a crystal structure is not outputted. Consequently, even if the techniques in PTL 1, PTL 2, and PTL 3 are used with the technique in NPL 1 above, determining a crystal structure will be difficult in cases where the number of arrangement combinations is huge.

Accordingly, an information processing system according to a first aspect of the present disclosure includes: an information processing apparatus that transmits information pertaining to a relational expression representing energy of a structure of an aggregate of atoms; and a computing apparatus that receives the information pertaining to the relational expression and performs computation using the relational expression. The aggregate of atoms includes states of two or more atoms. The relational expression is acquired on the basis of (a) atomic information pertaining to the states of the two or more atoms included in the aggregate and (b) position information pertaining to two or more positions where any of the states of the two or more atoms in the aggregate may be respectively located. The first relational expression includes two or more variables that indicate the states of atoms respectively located at the two or more positions. The information processing apparatus includes: a communicator that transmits the information pertaining to the relational expression to the computing apparatus, and receives, from the computing apparatus, values of the two or more variables derived by performing computation using the relational expression; and an outputter that outputs structure information indicating a structure of the aggregate that corresponds to the values of the two or more variables. Note that the aggregate of atoms is a crystal, a cluster, or a molecule, for example. Also, there is a one-to-one correspondence between the two or more positions and the two or more variables.

With this arrangement, a relational expression including two or more variables that indicate the states of atoms respectively located at two or more positions in an aggregate is used, and the values of the two or more variables in accordance with the magnitude of the energy represented by the relational expression are derived. That is, a search is carried out to find the structure of the aggregate in accordance with the magnitude of the energy. Such a relational expression may also be referred to as a function in Ising model form. Note that a function in Ising model form is also simply called an Ising model. Consequently, by using such a relational expression, an annealing machine can be applied to the search described above, and the computational costs associated with the search can be kept low compared to the technique of the related art that uses the cluster-expansion approach and a Monte Carlo method. Also, since there is a one-to-one correspondence between the values of the two or more variables and the two or more positions in the aggregate, once the values of the two or more variables are derived, a structure of an aggregate in accordance with the magnitude of the energy can be constructed easily. As a result, improved efficiency can be attained in the processing for searching for the structures of aggregates of atoms.

Note that an energy function is used as the function in Ising model form in the technique in PTL 3, too, but the energy function does not represent the energy of the structure (that is, the physical energy) of an aggregate of atoms in the present disclosure. In PTL 3, an empirical formula referred to as the tolerance factor, which serves as an indirect indicator of a stable structure, is used to construct an energy function for optimization by an Ising machine (also referred to as an annealing machine) as the function in Ising model form. This term "energy function" in PTL 3 is a customary term for an evaluation function to be optimized by an Ising machine, and does not represent the physical energy in the present disclosure.

An information processing apparatus according to a second aspect of the present disclosure includes: an acquirer that acquires a first relational expression including two or more variables that indicate states of atoms respectively located at two or more positions included in an aggregate of atoms as a relational expression representing energy of a structure of the aggregate, the first relational expression being acquired on the basis of (a) atomic information pertaining to the states of the two or more atoms included in the aggregate and (b) position information pertaining to two or more positions where any of the states of the two or more atoms in the aggregate may be respectively located; a communicator that transmits the information pertaining to the first relational expression to a computing apparatus that performs computation using the first relational expression, and receives, from the computing apparatus, values of the two or more variables derived by performing computation using the first relational expression; and an outputter that outputs structure information indicating a structure of the aggregate that corresponds to the values of the two or more variables.

With this arrangement, operations and effects similar to those of the information processing system described above can be exhibited by using a computing apparatus.

In an information processing apparatus according to a third aspect that depends from the second aspect, the computing apparatus may be an optimization apparatus that carries out a search to find an optimal solution to the first relational expression as the computation using the first relational expression.

With this arrangement, the values of the two or more variables that indicate the states of respective atoms are derived through energy optimization with respect to the first relational expression including the two or more variables. Consequently, an annealing machine can be used for energy optimization, and the computational costs associated with the energy optimization can be kept low. Also, since there is a one-to-one correspondence between the values of the two or more variables and the two or more positions in the aggregate, once the values of the two or more variables are derived, a stable structure of an aggregate can be constructed easily. As a result, improved efficiency can be attained in the processing for searching for stable structures of aggregates of atoms. Note that in the energy optimization, the energy represented by the relational expression is not necessarily minimized or optimized.

In an information processing apparatus according to a fourth aspect that depends from the second or third aspect, the states of the atoms may include at least one from among types of the atoms, spin states of the atoms, and atomic vacancies where the atoms are vacant.

With this arrangement, improved efficiency can be attained in the processing for searching for at least one from among types of atoms, spin states of atoms, and atomic vacancies included in a stable aggregate of atoms.

In an information processing apparatus according to a fifth aspect that depends from any one of the second to fourth aspects, the first relational expression may be represented by
[Math. 1]

$$E = A + \sum_i B_i \sigma_i + \sum_{i,j} C_{ij} \sigma_i \sigma_j \tag{1}$$

where E indicates energy of a structure of the aggregate, i and j each indicate the position in the aggregate, $\sigma_i$ is the variable at position i in the aggregate, $\sigma_j$ is the variable at position j in the aggregate, A is a zeroth-order cluster interaction coefficient, $B_i$ is a first-order cluster interaction coefficient, and $C_{ij}$ is a second-order cluster interaction coefficient. In an information processing apparatus according to a sixth aspect that depends from any one of the second to fourth aspects, the first relational expression may represent the energy by the sum of a first coefficient and n (where n is an integer equal to or greater than 2) terms, and among the n terms, the kth (where k is an integer equal to or greater than 1 and less than or equal to n) term may be the sum of the products respectively corresponding to groups of numbers that could be taken from the two or more variables, each of the groups containing k variables, and each of the products being the product of the k variables included in a group multiplied by a second coefficient corresponding to that group.

With this arrangement, an annealing machine can be used appropriately for energy optimization, and the computational costs can be kept low effectively.

In an information processing apparatus according to a seventh aspect that depends from any one of the second to fifth aspects, the acquirer may acquire the first relational expression by converting a second relational expression to an Ising model, the second relational expression being represented using the cluster-expansion approach on the basis of the atomic information and the position information.

With this arrangement, the first relational expression, namely an Ising model, is acquired by conversion from a second relational expression according to the cluster-expansion approach, thereby making it possible to apply the cluster-expansion approach and reduce the processing load associated with acquisition of the first relational expression.

In an information processing apparatus according to an eighth aspect that depends from the seventh aspect, the second relational expression may be represented by

[Math. 2]

$$E = \sum_{\alpha} V_{\alpha} \varphi_{\alpha} \qquad (2)$$

where E indicates energy of a structure of the aggregate, $\alpha$ is a cluster identifier, $V_{\alpha}$ is a coefficient indicating the magnitude of the contribution of cluster $\alpha$ to the energy, and $\phi_{\alpha}$ is a correlation function of cluster $\alpha$.

With this arrangement, more than one type of coefficient included in the first relational expression can be extracted from $V_{\alpha}$ and $\phi_{\alpha}$ included in Expression (2), and as a result, the first relational expression can be acquired appropriately.

An information processing apparatus according to a ninth aspect that depends from any one from among the second aspect and the fourth to eighth aspects, the computing apparatus may be a sampling apparatus that executes sampling based on a certain distribution as the computation using the first relational expression. The acquirer may further acquire distribution information pertaining to the certain distribution. The communicator may further transmit the distribution information to the sampling apparatus. In the receiving of the values of the two or more variables, the communicator may receive combinations from the sampling apparatus, each combination including values of the two or more variables, the combinations being derived by executing, with respect to the first relational expression, sampling based on the certain distribution corresponding to the distribution information. The outputter may output structure information indicating structures of the aggregate that correspond respectively to the combinations. For example, the certain distribution is a Boltzmann distribution, and the distribution information is information indicating the temperature used in the Boltzmann distribution. Note that the certain distribution is not limited to a Boltzmann distribution and may also be another distribution. Likewise, the distribution information is not limited to information indicating temperature and may be information of any kind, insofar as the information is for representing the certain distribution.

With this arrangement, combinations are derived by performing sampling based on a certain distribution with respect to the first relational expression including two or more variables that indicate the states of atoms respectively located at two or more positions in an aggregate. Note that sampling can also be referred to as searching. Also, the first relational expression may also be referred to as a function in Ising model form. Consequently, by using such a first relational expression, an annealing machine can be applied to the sampling, and the computational costs associated with the sampling can be kept low compared to the technique of the related art that uses the cluster-expansion approach and a Monte Carlo method. Also, since there is a one-to-one correspondence between the values of the two or more variables included in each of the combinations and the two or more positions in the aggregate, once the values of the two or more variables are derived, a structure of an aggregate can be constructed easily. As a result, improved efficiency can be attained in the processing for sampling or searching for the structures of aggregates of atoms.

In an information processing apparatus according to a 10th aspect that depends from any one from among the second aspect and the fourth to eighth aspects, the computing apparatus may include: an optimization apparatus that carries out a search to find an optimal solution to the first relational expression as the computation using the first relational expression; and a sampling apparatus that executes sampling based on a certain distribution as the computation using the first relational expression. The acquirer may further acquire distribution information pertaining to the certain distribution. In the transmitting of the information pertaining to the first relational expression, the communicator may transmit the information pertaining to the first relational expression to each of the optimization apparatus and the sampling apparatus, further receive, from the optimization apparatus, initial values of the two or more variables derived by carrying out a search to find an optimal solution to the first relational expression, and transmit the initial values of the two or more variables and the distribution information to the sampling apparatus. In the receiving of the values of the two or more variables, the communicator may receive combinations from the sampling apparatus, each combination including values of the two or more variables, the combinations being derived by executing, with respect to the first relational expression, sampling based on the initial values of the two or more variables and the certain distribution corresponding to the distribution information. The outputter may output structure information indicating structures of the aggregate that correspond respectively to the combinations.

With this arrangement, the values of the two or more variables derived by carrying out a search to find an optimal solution to the first relational expression, or in other words, by optimizing the energy of the first relational expression, are used as the initial values of the sampling based on the certain distribution. Consequently, it is possible to raise the likelihood of attaining an improvement in the processing speed and a reduction in the computational load of sampling.

In an information processing apparatus according to an 11th aspect that depends from any one from among the second aspect and the fourth to eighth aspects, the computing apparatus may include: an optimization apparatus that carries out a search to find an optimal solution to the first relational expression as the computation using the first relational expression; and a sampling apparatus that executes sampling based on a certain distribution as the computation using the first relational expression. The acquirer may further acquire distribution information pertaining to the certain distribution. In the transmitting of the information pertaining to the first relational expression, the communicator may transmit the information pertaining to the first relational expression to each of the optimization apparatus and the sampling apparatus, further transmit the distribution information to the sampling apparatus, receive combinations from the sampling apparatus, each combination including initial values of the two or more variables, the combinations being derived by executing, with respect to the first relational expression, sampling based on the certain distribution corresponding to the distribution information, and transmit the combinations to the optimization apparatus. In the receiving of the values of the two or more variables, the communicator may receive, from the optimization apparatus, values of the two or more variables derived by using the combinations to carry out a search to find an optimal solution to the first relational expression.

With this arrangement, combinations derived by performing sampling based on a certain distribution with respect to the first relational expression, that is, the values of the two or more variables included in each of the combinations, are used as the initial values for optimizing the energy of the first relational expression. Consequently, it is possible to raise the likelihood of attaining an improvement in the processing speed and a reduction in the computational load of sampling.

A computing apparatus according to a 12th aspect of the present disclosure includes: an arithmetic unit that derives values of two or more variables included in a relational expression by performing computation using the relational expression, the relational expression representing energy of a structure of an aggregate of atoms; and an outputter that outputs the values of the two or more variables derived by the arithmetic unit. The relational expression is acquired on the basis of (a) atomic information pertaining to the states of two or more atoms included in the aggregate and (b) position information pertaining to two or more positions where any of the states of the two or more atoms in the aggregate may be respectively located. The first relational expression includes the two or more variables that indicate the states of atoms respectively located at the two or more positions.

With this arrangement, the relational expression may also be referred to as a function in Ising model form, and thus by using such a relational expression, an annealing machine can be applied to the derivation of the values of the two or more variables, and the computational costs associated with the derivation can be kept low.

In a computing apparatus according to a 13th aspect that depends from the 12th aspect, the arithmetic unit may carry out a search to find an optimal solution to the relational expression as the computation using the relational expression.

With this arrangement, the computational costs associated with optimizing the energy of the relational expression can be kept low.

In a computing apparatus according to a 14th aspect that depends from the 12th aspect, the arithmetic unit may derive combinations by executing, with respect to the relational expression, sampling based on a certain distribution as the computation using the relational expression. The outputter may output the combinations. Each of the combinations may include values of the two or more variables included in the relational expression.

With this arrangement, the computational costs associated with the sampling based on a certain distribution with respect to the relational expression can be kept low.

Hereinafter, embodiments of the present disclosure will be described with reference to the drawings. The embodiments described hereinafter all illustrate preferred specific examples of the present disclosure. Consequently, features such as numerical values, shapes, materials, components, and the layout positions and connection configurations of the components indicated in the following embodiments are merely examples, and are not intended to limit the present disclosure. Thus, among the components in the following embodiments, components that are not described in the independent claim indicating the broadest concept are described as arbitrary or optional components.

Note that each diagram is a schematic diagram, and does not necessarily illustrate a strict representation. Also, in the drawings, structural elements that are substantially the same are denoted with the same signs, and duplicate description of such structural elements will be omitted or simplified.

(Embodiment 1)

[Configuration of information processing system 1000]

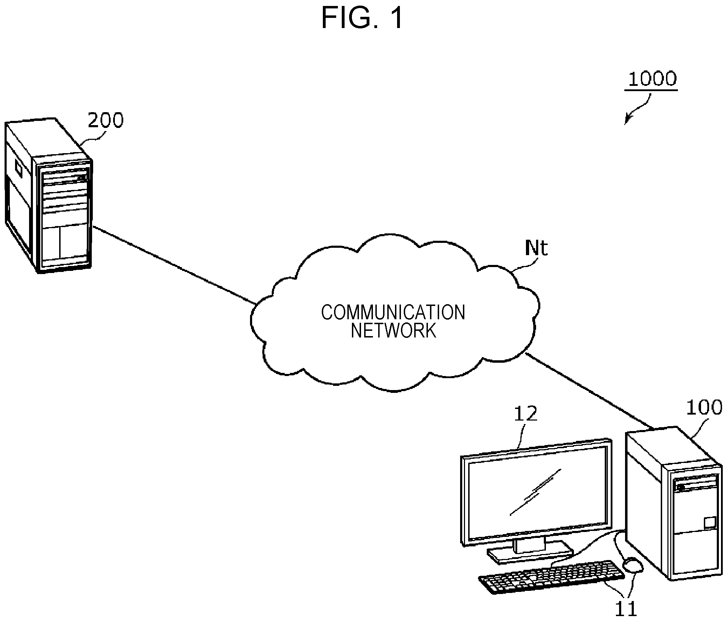

Fig. 1 is a diagram illustrating an example of a configuration of an information processing system 1000 according to the present embodiment.

The information processing system 1000 according to the present embodiment includes an information processing apparatus 100 and an optimization apparatus 200.

The information processing apparatus 100 is a computer for searching for stable structures of an aggregate of two or more atoms. Note that the aggregate of atoms may also be said to include the states of two or more atoms. Examples of the aggregate include crystals and clusters. The information processing apparatus 100 is connected to an input device 11 and a display device 12. The input device 11 is configured as a keyboard, a touch sensor, a touchpad, or a mouse, for example, and outputs to the information processing apparatus 100 a signal in accordance with an input operation performed by a user. The display device 12 is a liquid crystal display, a plasma display, or an organic electroluminescence (EL) display, for example, and displays an image on the basis of an image signal outputted from the information processing apparatus 100. Such an information processing apparatus 100 acquires a first relational expression as an Ising model relational expression representing the energy of the aggregate. The information processing apparatus 100 transmits information pertaining to the first relational expression, which represents the energy of the structure of the aggregate of two or more atoms, to the optimization apparatus 200 over a communication network Nt. Additionally, the information processing apparatus 100 acquires from the optimization apparatus 200 the values of variables that, for example, minimize the energy represented by the first relational expression. Each of the variables is a variable included in the first relational expression. The information processing apparatus 100 determines the stable structure described above according to the values of the variables. The information processing apparatus 100 may output structure information indicating the structure determined in this way to an external apparatus or device, and may also output the structure information as an image signal to the display device 12 to display the structure on the display device 12.

The optimization apparatus 200 is a computer that communicates with the information processing apparatus 100 over the communication network Nt such as the Internet, and optimizes (that is, optimizes the energy of) the first relational expression described above. That is, the optimization apparatus 200 receives information pertaining to the first relational expression from the information processing apparatus 100 over the communication network Nt and carries out a search to find an optimal solution to the first relational expression. Through this searching, the energy represented by the first relational expression is, for example, minimized. Note that in this searching, or in other words energy optimization, the energy represented by the first relational expression is not necessarily minimized or optimized, but the present embodiment is described as though the energy is minimized. The optimization apparatus 200 then derives the values of the variables with which the energy represented by the first relational expression is the lowest.

[Configuration of information processing apparatus and optimization apparatus]

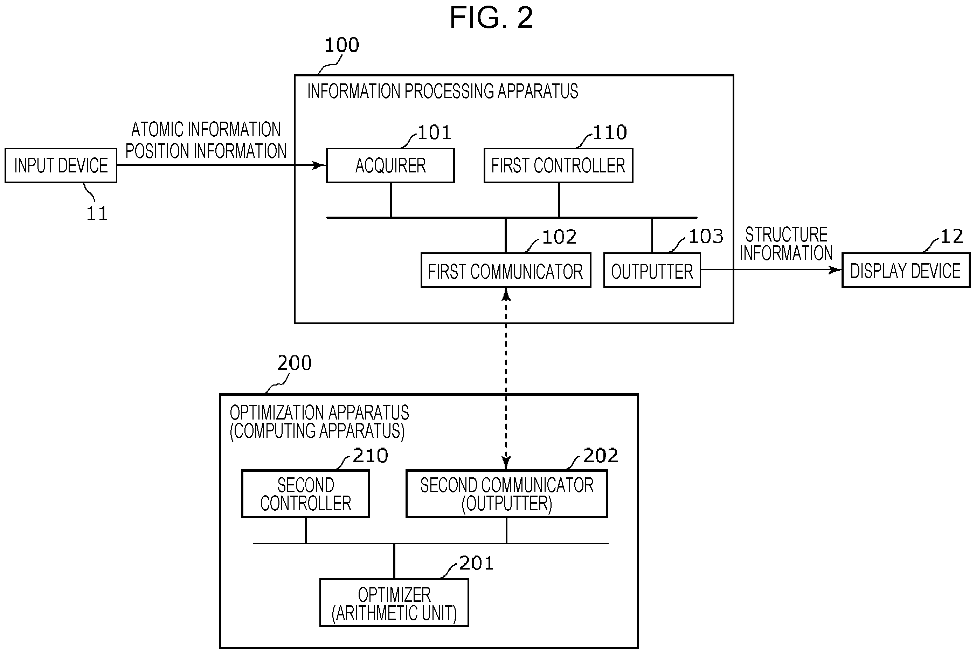

Fig. 2 is a block diagram illustrating an example of a functional configuration of the information processing apparatus 100 and the optimization apparatus 200.

The information processing apparatus 100 includes an acquirer 101, a first communicator 102, an outputter 103, and a first controller 110.

The acquirer 101 acquires the first relational expression described above on the basis of atomic information and position information. For example, the acquirer 101 acquires the atomic information and position information as a signal outputted from the input device 11. Note that the acquirer 101 may also read atomic information and position information stored in a recording medium.

The atomic information is information pertaining to the states of two or more atoms included in an aggregate of atoms. The atomic information includes at least one from among types of atoms, spin states of atoms, and atomic vacancies where atoms are vacant, for example. Spin is electronic spin or nuclear spin, for example. Note that the present embodiment is described as though the atomic information indicates types of atoms. Moreover, the atomic information may indicate the types of atoms directly or indirectly. For example, if the aggregate is a set of Cu (i.e., copper) atoms and Au (i.e., gold) atoms, the atomic information indicates Cu and Au. The atomic information may not only indicate the types of the atoms, but also indicate the spin states of the atoms. The position information is information pertaining to two or more positions where any of the states of two or more atoms in the aggregate may be respectively located. Note that the position information may also indicate the number of the positions, and may also indicate the positions directly or indirectly. Such positions may also be referred to as sites or site positions. The first relational expression includes two or more variables that indicate the states of atoms respectively located at two or more such positions. Also, there is a one-to-one correspondence between the two or more positions and the two or more variables. Note that an aggregate of atoms may also include other information unrelated to the information pertaining to the states of two or more atoms and the information pertaining to the two or more positions thereof.

The first communicator 102 is a device that communicates with the optimization apparatus 200, and transmits information pertaining to the first relational expression acquired by the acquirer 101 to the optimization apparatus 200. Furthermore, the first communicator 102 receives from the optimization apparatus 200 the respective values of the two or more variables derived by the optimization apparatus 200 with respect to the first relational expression obtained from the transmitted information. The values of the two or more variables are derived by the energy optimization described above, namely by carrying out a search to find an optimal solution to the first relational expression. In this way, the first communicator 102 transmits information pertaining to the first relational expression to the optimization apparatus 200, and receives from the optimization apparatus 200 the values of two or more variables derived by carrying out a search to find an optimal solution to the first relational expression.

The outputter 103 outputs structure information that indicates the structure of the aggregate described above, corresponding to the values of the two or more variables derived by the optimization apparatus 200. For example, the outputter 103 outputs the structure information as an image signal to the display device 12, and thereby displays the structure of the aggregate on the display device 12.

The first controller 110 controls the acquirer 101, the first communicator 102, and the outputter 103, and also executes processing other than the processing by these components.

The optimization apparatus 200 is an example of a computing apparatus that receives information pertaining to the first relational expression and performs computation using the first relational expression, and includes an optimizer 201, a second communicator 202, and a second controller 210.

The second communicator 202 is a device that communicates with the information processing apparatus 100, and receives the information pertaining to the first relational expression described above from the information processing apparatus 100. Additionally, the second communicator 202 transmits to the information processing apparatus 100 the values of two or more variables derived by the optimizer 201. That is, the second communicator 202 functions as an outputter that outputs the values of the two or more variables derived by the optimizer 201.

The optimizer 201 acquires the information pertaining to the first relational expression described above from the information processing apparatus 100 via the second communicator 202, and optimizes the energy of the first relational expression obtained from the information. That is, the optimizer 201 derives the values of the two or more variables included in the first relational expression by carrying out a search to find an optimal solution to the first relational expression, which represents the energy of the structure of an aggregate of atoms. Note that the optimizer 201 according to the present embodiment is an example of an arithmetic unit, and derives the values of the two or more variables included in the first relational expression by performing computation using the first relational expression, which represents the energy of the structure of an aggregate of atoms. In the present embodiment, the optimizer 201 carries out a search to find an optimal solution to the first relational expression as the computation using the first relational expression.

The second controller 210 controls the optimizer 201 and the second communicator 202, and also executes processing other than the processing by these components.

Each of the information processing apparatus 100 and the optimization apparatus 200 as above may be configured by a processor such as a central processing unit (CPU), volatile memory and non-volatile memory, and a program stored in the non-volatile memory, for example. In this case, the functional configuration of each of the information processing apparatus 100 and the optimization apparatus 200 is realized by having the processor execute the program.

[Details of processing by information processing system 1000]

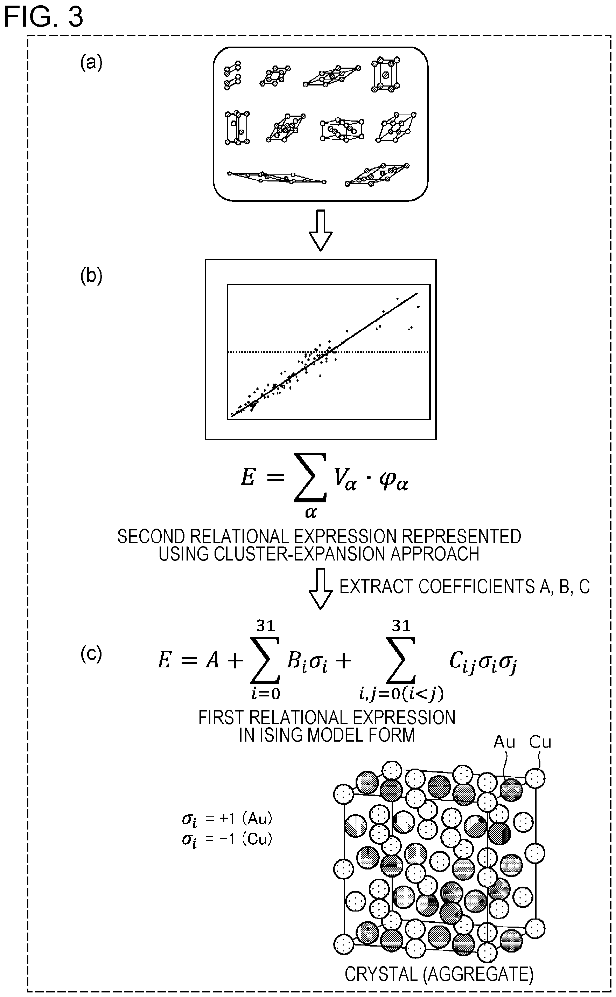

Fig. 3 is a diagram for explaining an example of processing by the information processing apparatus 100.

As illustrated in Fig. 3(a), the first controller 110 of the information processing apparatus 100 uses first-principles computation to calculate the energy of each of more than one crystal structure. These crystal structures are structures with a small number of sites to keep the energy calculation load small. For example, the number of sites is 16 or less. For the energy calculation, an analysis tool such as the Alloy Theoretic Automated Toolkit (ATAT) or the Vienna Ab initio Simulation Package (VASP) may be used, for example.

Next, the acquirer 101 acquires atomic information and position information from the input device 11, and as illustrated in Fig. 3(b), generates an energy prediction model corresponding to the atomic information and the position information on the basis of the relationship between the crystal structures and their calculated energies. For example, the atomic information indicates Cu and Au, and the position information indicates 32 sites. That is, the energy prediction model is a model for predicting the energy of a crystal structure which has 32 sites and in which Cu or Au is located at each of the sites. The energy prediction model is generated as a second relational expression indicated by the following Expression (2) through machine learning based on the relationship between the crystal structures and their calculated energies, for example.

[Math. 3]

$$E = \sum_{\alpha} V_{\alpha} \varphi_{\alpha} \qquad (2)$$

In Expression (2), E represents the energy of a crystal structure, and $\alpha$ is a cluster identifier. A cluster is a group of atoms included in a crystal, and any one cluster among the clusters included in the crystal is identified by $\alpha$. Also, $V_{\alpha}$ is a coefficient indicating the magnitude of the contribution of cluster $\alpha$ to the energy E. Also, $\phi_{\alpha}$ is a correlation function of cluster $\alpha$. That is, the acquirer 101 generates the second relational expression, which is represented using the cluster-expansion approach, on the basis of the atomic information and the position information. In other words, the acquirer 101 computes effective cluster interactions (ECIs) of a cluster expansion up to a certain order. Note that

[Math. 6]

$$\varphi$$

in the diagram and expressions may be denoted as $\phi$ in locations other than the diagrams and expressions.

Next, as illustrated in Fig. 3(c), the acquirer 101 acquires the first relational expression by converting the second relational expression to an Ising model. That is, the first relational expression is an Ising model, and is represented by Expression (1) below, for example.

[Math. 4]

$$E = A + \sum_{i=0}^{31} B_i \sigma_i + \sum_{i,j=0(i<j)}^{31} C_{ij} \sigma_i \sigma_j \qquad (1)$$

In Expression (1), E indicates the energy of a crystal structure, while i and j each indicate a position (that is, a site position) in the crystal. Also, $\sigma_i$ is a variable indicating the type of atom located at the position i in the crystal, and $\sigma_j$ is a variable indicating the type of atom located at the position j in the crystal. Here, $\sigma_i$ and $\sigma_j$ represent only the difference between the position i and the position j, and mean the same as variables. Note that these variables are also referred to as Ising variables. Also, A is a zeroth-order cluster interaction coefficient, Bi is a first-order cluster interaction coefficient, and $C_{ij}$ is a second-order cluster interaction coefficient. Note that these interaction coefficients are hereinafter also simply referred to as coefficients. Specifically, when acquiring the first relational expression as in Expression (1) by conversion to an Ising model, the acquirer 101 acquires the first relational expression in Expression (1) by extracting the coefficients A, Bi, and $C_{ij}$ from the second relational expression in Expression (2).

Clusters such as zeroth-order clusters, first-order clusters, and second-order clusters are groups of atoms, and the order number is the number of atoms included in the group. For example, a second-order cluster may be a group of two adjacent atoms included in the crystal, and may also be a group of two atoms apart from each other. That is, a second-order cluster is a combination of any two atoms included in the crystal. Also, in one specific example, if the atom at the position i is Au, the variable $\sigma_i$ corresponding to that position i has a value of +1, whereas if the atom at the position i is Cu, the variable $\sigma_i$ corresponding to that position i has a value of -1. In this way, the variable $\sigma_i$ indicates the type of atom located at the position i with the two values of +1 and -1. Note that the variable $\sigma_i$ may indicate not only the type of an atom but also the spin state of the atom.

The first relational expression expresses the energy E by the sum of a zeroth-order term, a first-order term, and a second-order term, as in Expression (1). That is, in Expression (1), the energy E is expressed by terms up to the second order. However, the order number is not limited to the second order and may also be the third order or higher. For example, the first relational expression may also express the energy E by the sum of a zeroth-order term, a first-order term, a second-order term, and so on up to an Nth-order (where N is an integer equal to or greater than 3) term. Note that the acquirer 101 may also acquire a first relational expression with terms up to the third order or higher, and thereafter round that first relational expression into a first relational expression with terms up to the second order.

In other words, the first relational expression represents the energy E by the sum of a first coefficient A and n (where n is an integer equal to or greater than 2) terms. Among the n terms, the kth (where k is an integer equal to or greater than 1 and less than or equal to n) term is the sum of the products respectively corresponding to the groups of numbers that could be taken from two or more variables $\sigma$. each of the groups contains k variables $\sigma$, and the product thereof is the product of the k variables $\sigma$ included in that group multiplied by a second coefficient ($B_i$ or $C_{ij}$, for example) corresponding to that group. Note that k corresponds to the order number described above, and the order number is not limited to the second order and may also be the third order or higher.

Expression (1) is an example of the first relational expression for the case of a crystal structure having 32 sites, with i and j each indicating an integer from 0 to 31. However, the number of sites in a crystal structure is not limited to 32, and may also be 33 or more. In this case, i and j in Expression (1) each indicate an integer from 0 to (m-1) (where m is the number of sites).

In this way, in the present embodiment, energy optimization is performed using the first relational expression, which is a function in Ising model form, like Expression (1) for example. Consequently, an annealing machine can be used appropriately for the energy optimization, and the computational costs can be kept low effectively.

Also, in the present embodiment, the first relational expression, namely an Ising model, is acquired by conversion from a second relational expression according to the cluster-expansion approach, thereby making it possible to apply the cluster-expansion approach and reduce the processing load associated with acquisition of the first relational expression. Furthermore, in the present embodiment, more than one type of coefficient included in the first relational expression can be extracted from $V_\alpha$ and $\phi_\alpha$ included in the second relational expression, like Expression (2) for example, and as a result, the first relational expression can be acquired appropriately.

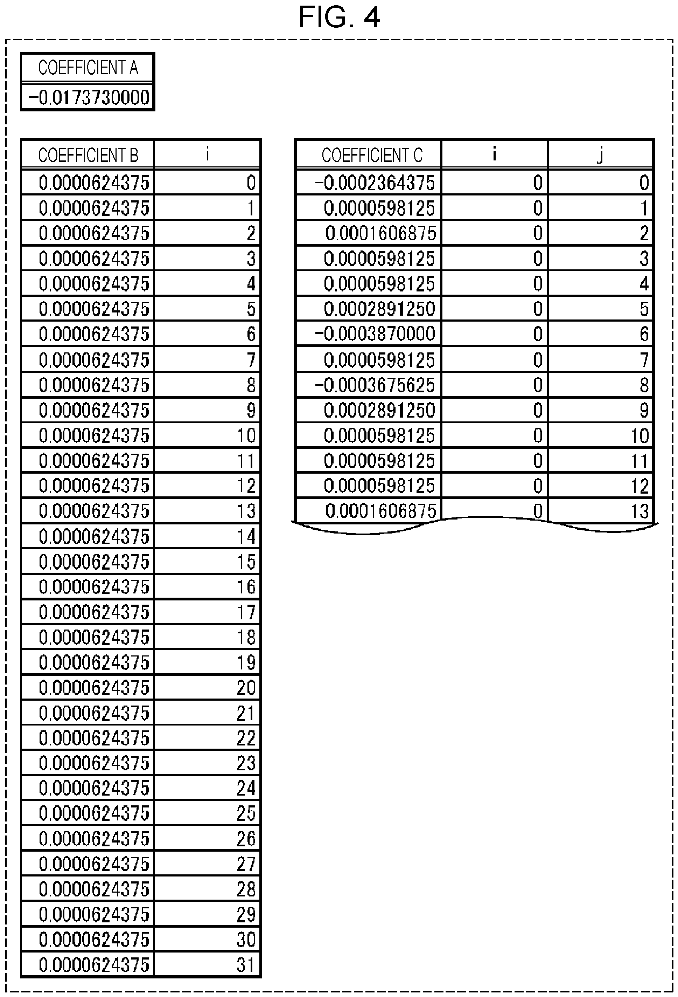

Fig. 4 is a diagram illustrating an example of coefficients obtained by the information processing apparatus 100.

The coefficient A is the zeroth-order cluster interaction coefficient as described above, and as illustrated in Fig. 4, is -0.0173730000 for example. The coefficient $B_i$ is the first-order cluster interaction coefficient as described above, and in the case of an aggregate with 32 sites, indicates a numerical value for each position i=0 to 31. The coefficient $C_{ij}$ is the second-order cluster interaction coefficient as described above, and in the case of an aggregate with 32 sites, indicates a numerical value for all combinations of each position i=0 to 31 and each position j=0 to 31, under the condition that i<j. The information processing apparatus 100 acquires the first relational expression by extracting such coefficients A, Bi, and $C_{ij}$ from the second relational expression, and transmits the coefficients as information pertaining to the first relational expression to the optimization apparatus 200. Note that the information processing apparatus 100 may transmit not only the coefficients A, Bi, and $C_{ij}$, but also atomic information and position information. Constraint conditions on the atomic information may also be transmitted (fixing a composition ratio to certain values, for example). The entire first relational expression may also be transmitted to the optimization apparatus 200.

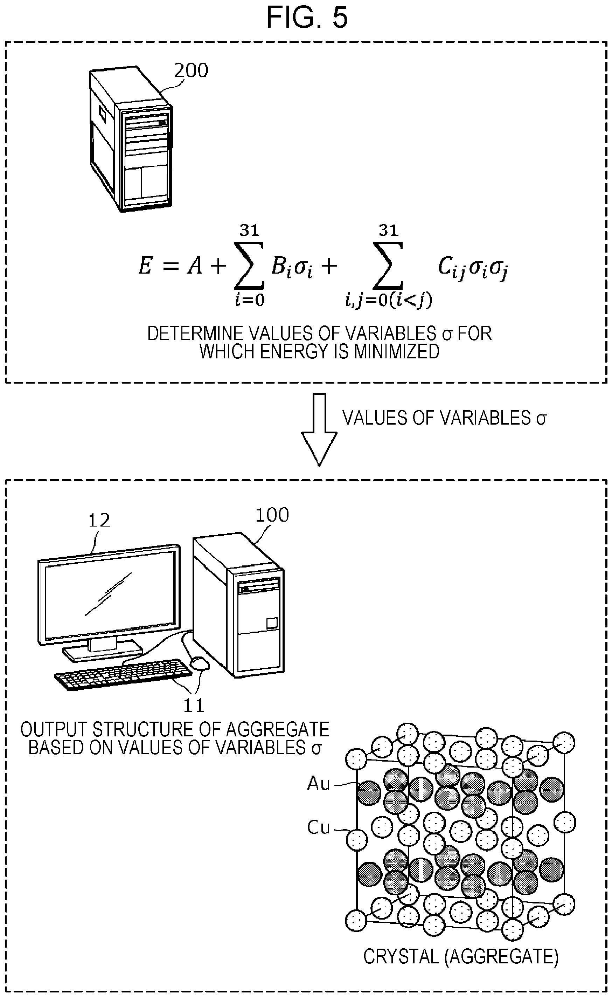

Fig. 5 is a diagram for explaining an example of processing by the optimization apparatus 200 and the information processing apparatus 100.

The optimization apparatus 200 receives the coefficients A, Bi, and $C_{ij}$ transmitted from the information processing apparatus 100. At this time, the optimization apparatus 200 may receive not only the coefficients, but also atomic information and position information from the information processing apparatus 100. The optimization apparatus 200 then constructs the first relational expression on the basis of the coefficients, information, and the like, and derives the values of the variables $\sigma$ (here, $\sigma_i$) that minimize the energy E represented by the first relational expression. In other words, the optimization apparatus 200 determines the values of the variables $\sigma_i$ through energy optimization. Note it can be said that when the number of sites is 32, that is, when the number of integers indicated by i is 32, the number of values of the variables $\sigma$ is also 32. At this time, the optimization apparatus 200 performs the above energy optimization by using an annealing machine, which is a computer specialized in the optimization of Ising models. The annealing machine may be a quantum annealing machine, a quantum-inspired annealing machine, or the like. A gate-based quantum computer may also be used instead of an annealing machine. In the gate-based quantum computer, the quantum approximate optimization algorithm (QAOA) may be used. The optimization apparatus 200 then transmits the derived or determined values of the variables $\sigma_i$ to the information processing apparatus 100. As an example, when the energy E represented by the first relational expression is minimized by the optimization apparatus 200, E=-490.08($\times 10^{-4}$). In this case, the derived values of the variables $\sigma_i$ are $\sigma_i$=-1 (for i=0 to 7 and 16 to 23) and $\sigma_i$=+1 (for i=8 to 15 and 24 to 31), for example.

The information processing apparatus 100 constructs a structure of an aggregate of atoms (a crystal structure, for example) on the basis of the values of the variables $\sigma_i$ transmitted from the optimization apparatus 200. That is, the information processing apparatus 100 constructs a structure of an aggregate by placing an atom of the type corresponding to the value of the variable $\sigma_i$ at the position i in the aggregate. For example, in the construction of a structure for the case of $\sigma_i$=-1 (for i=0 to 7 and 16 to 23) and $\sigma_i$=+1 (for i=8 to 15 and 24 to 31), an Au or Cu atoms is placed at each position. The information processing apparatus 100 then outputs structure information indicating the constructed structure of the aggregate. Outputting the structure information causes the structure of the aggregate to be displayed on the display device 12, for example.

[Flow of processing]

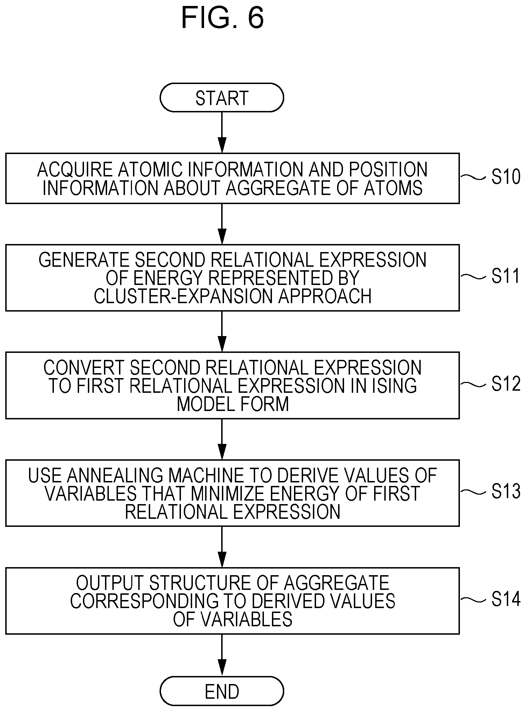

Fig. 6 is a flowchart illustrating an example of processing operations by the information processing system 1000.

(Step S10)

The acquirer 101 of the information processing apparatus 100 acquires atomic information and position information about an aggregate of atoms.

(Step S11)

The acquirer 101 generates the second relational expression of energy represented by the cluster-expansion approach.

(Step S12)

The acquirer 101 converts the second relational expression to the first relational expression in Ising model form. That is, the acquirer 101 acquires the first relational expression by converting the second relational expression to an Ising model.

(Step S13)

The optimizer 201 of the optimization apparatus 200 uses an annealing machine to derive the values of the variables in the first relational expression that minimize the energy of the first relational expression. For example, the first controller 110 of the information processing apparatus 100 causes the optimization apparatus 200 to execute processing to minimize the energy (that is, optimize the energy) of the first relational expression by causing information pertaining to the first relational expression to be transmitted from the first communicator 102 to the optimization apparatus 200. Note that it can also be said that the first controller 110 of the information processing apparatus 100 performs energy optimization using the optimization apparatus 200.

(Step S14)

The outputter 103 of the information processing apparatus 100 constructs a structure of an aggregate on the basis of the derived values of the variables, and outputs structure information indicating the structure. For example, the second controller 210 of the optimization apparatus 200 causes the derived values of the variables to be transmitted from the second communicator 202 to the information processing apparatus 100. The outputter 103 constructs a structure of an aggregate using the values of the variables transmitted from the optimization apparatus 200.

As above, in the present embodiment, to obtain a stable structure of an aggregate, the first relational expression including two or more variables that indicate the states of atoms respectively located at two or more positions in an aggregate is used, and the values of the two or more variables with which the energy represented by the first relational expression is, for example, the lowest are derived. That is, the values of the two or more variables that indicate the states of respective atoms are derived through energy optimization with respect to the first relational expression including the two or more variables. Such a first relational expression may also be referred to as a function in Ising model form. Consequently, by using such a first relational expression, an annealing machine can be applied to the energy optimization, and the computational costs associated with the energy optimization can be kept low compared to the technique of the related art that uses the cluster-expansion approach and a Monte Carlo method. Also, since there is a one-to-one correspondence between the values of the two or more variables and the two or more positions in the aggregate, once the values of the two or more variables are derived, a stable structure of an aggregate can be constructed easily. As a result, improved efficiency can be attained in the processing for searching for stable structures of aggregates of atoms.

Note that in the present embodiment, a search to find an optimal solution to the first relational expression is carried out as computation using the first relational expression, but computation other than such a search may also be performed. Even in such cases, in the present embodiment, the values of two or more variables in accordance with the magnitude of the energy represented by the first relational expression are derived. That is, a search is carried out to find the structure of the aggregate in accordance with the magnitude of the energy. Moreover, by using the first relational expression, an annealing machine can be applied to the search described above, and the computational costs associated with the search can be kept low. Also, once the values of the two or more variables are derived, a structure of an aggregate in accordance with the magnitude of the energy can be constructed easily. As a result, improved efficiency can be attained in the processing for searching for the structures of aggregates of atoms.

[Processing in step S11]

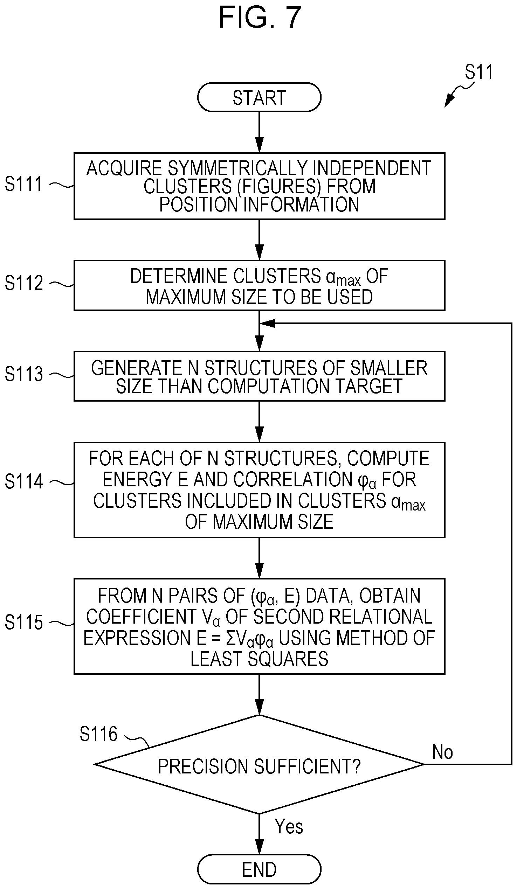

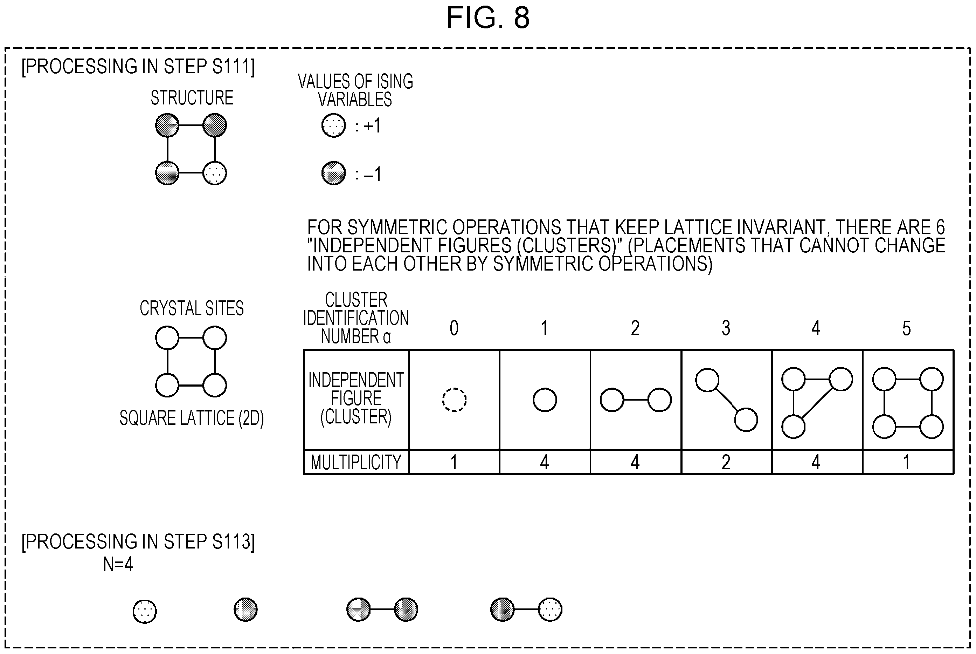

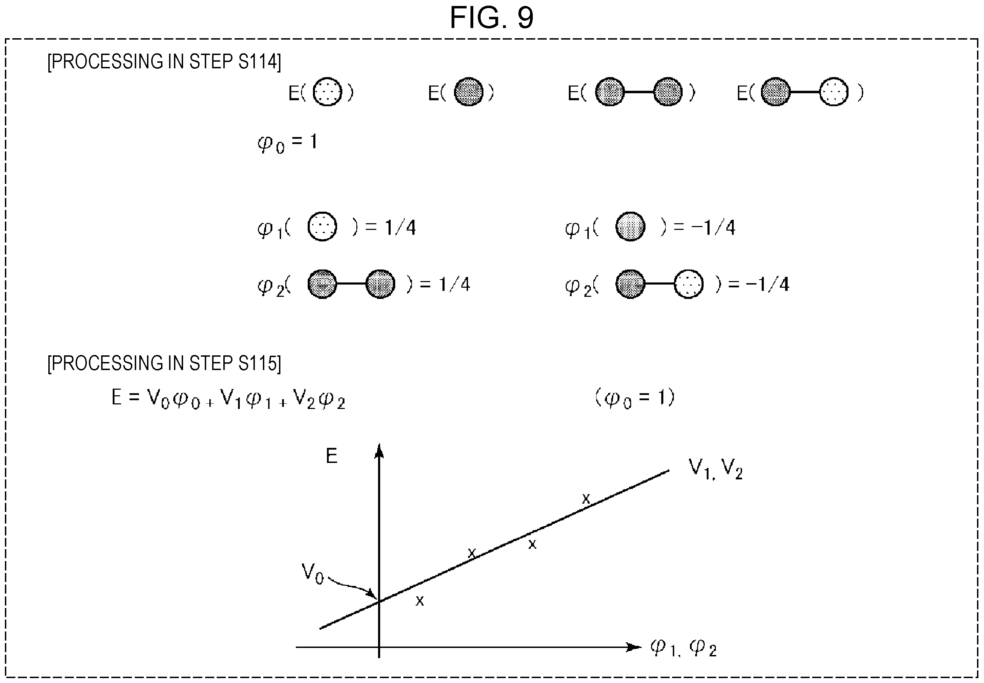

Hereinafter, the processing in step S11 of Fig. 6 will be described in detail using Figs. 7-9. Note that Fig. 7 is a flowchart illustrating in detail an example of the processing in step S11 of Fig. 6. Fig. 8 is a diagram illustrating a specific example of the processing in steps S111 and S113 of Fig. 7, and Fig. 9 is a diagram illustrating a specific example of the processing in steps S114 and S115 of Fig. 7.

As illustrated in Fig. 7, first, from position information about an aggregate of atoms, the acquirer 101 acquires symmetric transformations that keep the position information invariant, and acquires independent clusters (figures) for the symmetric transformations (step S111). From among such clusters, the acquirer 101 determines clusters $\alpha_{max}$ of maximum size to be used to generate the second relational expression (step S112). Next, the acquirer 101 generates multiple (designated as N) structures of smaller size than the computation target (step 5113). For each of the N structures, the acquirer 101 uses first-principles computation to compute the energy E and a correlation $\phi_\alpha$ for cluster groups (sub-clusters) $\alpha$ included within the clusters $\alpha_{max}$ of maximum size (step S114). From the N pairs of ($\phi_\alpha$, E) data generated in this way, the acquirer 101 calculates the coefficient $V_\alpha$ of the second relational expression using the method of least squares (step S115). The acquirer 101 then determines whether or not the coefficient $V_\alpha$ is sufficiently precise (step S116). If the coefficient $V_\alpha$ obtained in the method of least squares is not sufficiently precise (step S116, No), the acquirer 101 may add structures to increase the data. Precision is evaluated by cross-validation, for example. More specifically, when calculating the coefficient $V_\alpha$ of the second relational expression using an analysis tool such as the ATAT, the acquirer 101 determines that the precision is sufficient when the cross-validation score is less than or equal to 0.05. If the acquirer 101 determines that the precision is sufficient (step S116, Yes), the processing in step S11 ends.

Figs. 8 and 9 illustrate the processing in step S11 specifically using simple two-dimensional structures (see NPL (INUI, Haruyuki (Ed.), High-Entropy Alloys-Various New Properties Generated by the Cocktail Effect, Uchida Rokakuho, 01 May 2020)).

As in [Processing in step S111] of Fig. 8, consider using the cluster-expansion approach to obtain the energy of a structure in which atomic species are placed on a square lattice with four sites. For clusters generated by choosing several of the sites in the square lattice, the clusters which are independent with respect to symmetric transformations that keep the square lattice invariant are the six types in Fig. 8. Note that in the table in Fig. 8, the number of figures that change into each other under symmetric transformations is referred to and denoted as the multiplicity. Among these, the figure formed by "not choosing a site" (this is referred to as the zero cluster) is also included out of convenience, and is denoted by the cluster identification number $\alpha=0$. Thereafter, identification numbers from 1 to 5 are assigned as in Fig. 8 in order of increasing cluster size. In the current specific example, $\alpha_{max}$ is set to two, that is, cluster expansion is performed using the figures formed by two sites in immediate proximity as the clusters of maximum size. The cases of $\alpha=0$, 1 are also all considered as sub-clusters. The following considers the case of N=4, taking the structures as illustrated in [Processing in step 5 113] of Fig. 8 as data. For these four structures, the acquirer 101 computes the respective energies and $\phi_0$, $\phi_1$, and $\phi_2$. The value of the zero cluster is defined to be 1 for all structures. Several computational results are illustrated in [Processing in step S114] of Fig. 9. Finally, the acquirer 101 uses the method of least square to obtain a linear equation that approximates the correlation-energy data points of these four groups. A conceptual diagram is illustrated in [Processing in step S115] of Fig. 9. This means that the coefficients $V_0$, Vi, and $V_2$ of the linear equation E = $V_0\phi_0$ + $V_1\phi_1$ + $V_2\phi_2$ are determined, and the second relational expression is obtained.

[Processing in step S 12]

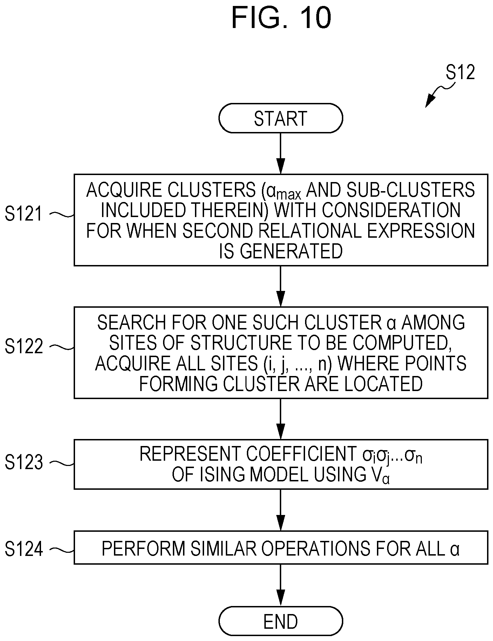

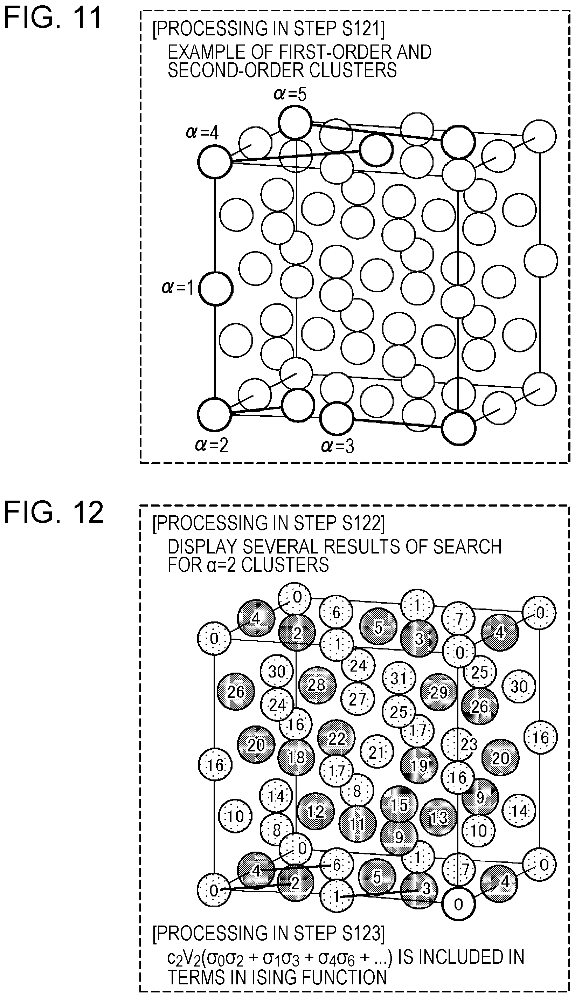

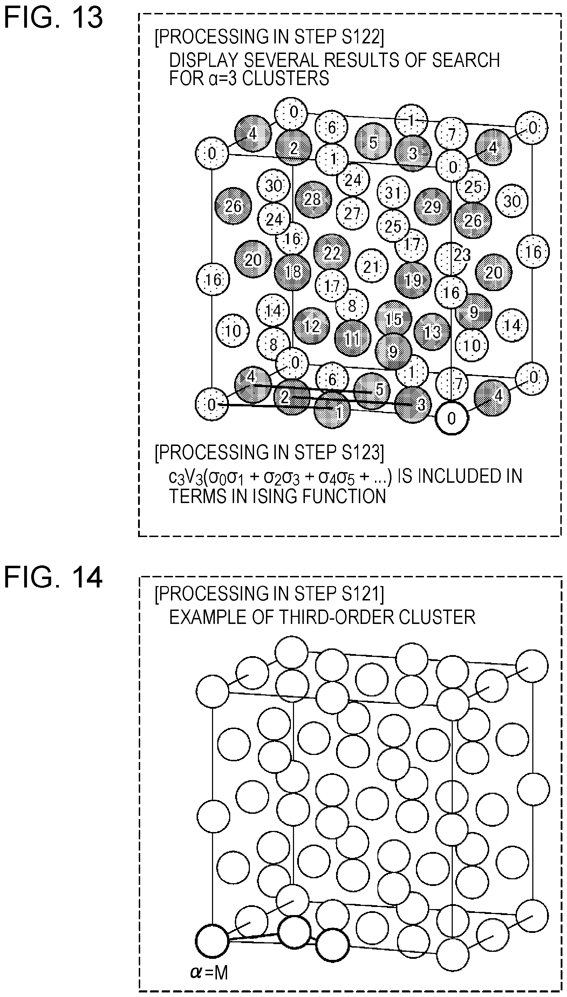

Hereinafter, the processing in step S12 of Fig. 6 will be described in detail using Figs. 10-15. Note that Fig. 10 is a flowchart illustrating in detail an example of the processing in step S12 of Fig. 6. Figs. 11 and 14 are diagram illustrating specific examples of the processing in steps S 121 of Fig. 10, while Figs. 12, 13, and 15 are diagram illustrating specific examples of the processing in steps S122 and S123 of Fig. 10.

As illustrated in Fig. 10, first, the acquirer 101 acquires clusters with consideration for when the second relational expression is generated (step S121). Note that these clusters are the clusters $\alpha_{max}$ of maximum size and the cluster groups (sub-clusters) included therein. For one such cluster $\alpha$, the acquirer 101 searches for a group of sites matching the cluster $\alpha$ from the sites of the structure to be computed, and acquires all groups of sites returned by the search (step S122). Assuming that the groups of sites are enumerated by site numbers (i, j, ..., n), the coefficient of the $\sigma_i\sigma_j \dots \sigma_n$ term of the function in Ising form is $c_\alpha V_\alpha$ (where $c_\alpha$ is typically a constant based on $\alpha$). That is, the acquirer 101 uses $V_\alpha$ to represent the coefficient of $\sigma_i\sigma_j...\sigma_n$ in the function in Ising form (step S123). Note that $\sigma_i\sigma_j...\sigma_n$ represents the product of the Ising variables respectively indexed by the group of site numbers (i, j, ..., n). The acquirer 101 performs this procedure for all $\alpha$, and thereby determines the coefficients of the function in Ising form that represents the energy of the structure to be computed (step S124).

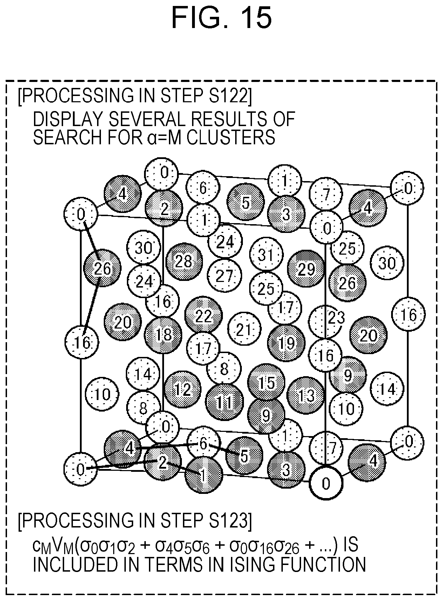

Figs. 11-15 illustrate portions of the processing in step S12 specifically using the structure of an Au-Cu alloy with 32 sites. Several first-order and second-order clusters are illustrated in [Processing in step S121] of Fig. 11. The cluster of $\alpha=2$ is a figure formed from two sites in immediate proximity. [Processing in step S122] of Fig. 12 partially illustrates where these clusters of $\alpha=2$ are located in the alloy structure. [Processing in step S123] of Fig. 12 indicates the term and coefficient (namely, $c_2V_2$) in the function in Ising form that correspond to the cluster at the sites illustrated in [Processing in step S122]. In Fig. 13, [Processing in step S122] illustrates an example of positions occupied by the cluster of $\alpha=3$ in the alloy structure, and [Processing in step S123] indicates the term and coefficient (namely, $c_3V_3$) in the function in Ising form that correspond to the cluster at the sites illustrated in [Processing in step S122]. An example of a third-order cluster (cluster formed from three points) is illustrated in [Processing in step S121] of Fig. 14. In Fig. 15, [Processing in step S122] illustrates an example of positions occupied by the cluster of $\alpha=M$ (where M is a positive integer and the identification number of the cluster illustrated in Fig. 14) in the alloy structure, and [Processing in step S123] indicates the term and coefficient (namely, $c_MV_M$) in the function in Ising form that correspond to the cluster at the sites illustrated in [Processing in step S122]. The coefficients obtained by such processing in step S123 are used as the coefficients of the first relational expression.

<Supplement 1>

In Embodiment 1, a function represented using the cluster-expansion approach (namely, the second relational expression) is converted to a function in Ising model form (namely, the first relational expression), and an annealing machine is applied to the function in Ising model form. The following reasons have been behind the lack of motivation to apply annealing machines to functions represented using the cluster-expansion approach heretofore.

A first reason is that arithmetic processing of functions in the cluster-expansion approach is faster than arithmetic processing of functions in Ising model form when using conventional classical algorithms or classical computers. Note that a function in the cluster-expansion approach is, for example, the second relational expression described above, which is represented in the form of the sum of the products of effective cluster interactions (ECIs) and correlation functions. Accordingly, taking the trouble to convert the second relational expression to a function in Ising model form has been considered unnecessary.

A second reason is that optimization by annealing machines is a metaheuristic approach, and it has been unclear whether good solutions could be obtained effectively as compared to functions in the cluster-expansion approach.

A third reason is that the number of bits that annealing machines can process has been small, and annealing machines have been unsuited to aggregates with a large number of atoms.

These reasons are overturned as follows.

In regard to the first reason, it has become clear that if the second relational expression is converted to the first relational expression, which is a function in Ising model form, and an annealing machine is applied to the first relational expression as in Embodiment 1, the arithmetic processing using the annealing machine is fast. That is, it has become clear that the total time, including the time to convert the second relational expression to the first relational expression and the time taken by the arithmetic processing using the annealing machine, is shorter than time taken by arithmetic processing using classical algorithms on functions in the cluster-expansion approach.

In regard to the second reason, actual verification has made it clear that optimization by annealing machines can also be used to obtain good solutions efficiently.

In regard to the third reason, recent advances in computer processing technology have led to an increase in the number of bits that annealing machines can process compared to the past.

Consequently, based on these matters which have become clear, a significant effect can be obtained with Embodiment 1.

<Supplement 2>

Arithmetic processing using an annealing machine is faster than arithmetic processing using classical algorithms. The benchmark conditions for a mathematical optimization solver, that is, classical algorithms, are given below.

- Gurobi Optimizer® (GUROBI®) ver. 9.12 (64-bit)
- Windows® 10
- CPU: Intel® Xeon® Gold 6140 (Skylake cores), memory: 64 GB
- Thread count: 32 threads (36 physical cores, 72 logical processors)

For cases where the number of bits is n=32 and n=256, the time for arithmetic processing using classical algorithms is less than 1 second, whereas the time for arithmetic processing using the annealing machine is less than or equal to 0.5 seconds. Note that the number of bits n corresponds to the number of atoms in an aggregate to be processed. On the other hand, in the case where the number of bits is n=2048, the time for arithmetic processing using classical algorithms is 50 to 270 minutes, whereas the time for arithmetic processing using the annealing machine is less than or equal to 1 second. Note that the function used in the measurement of the time for arithmetic processing described above, that is, the function corresponding to the aggregate to be processed, is a function in Ising model form for classical algorithms, too, in the same way as for the annealing machine. That is, the time for arithmetic processing of the same function in Ising model form is measured for each of the classical algorithms and the annealing machine.

Consequently, arithmetic processing using the annealing machine is superior to arithmetic processing using classical algorithms for large-scale problems, such as when the set to be processed has a large number of atoms.

In the case where the composition ratio of atomic species included in the aggregate is fixed and the number of bits is n=2048, the average processing speed for each composition ratio is 3300 seconds for classical algorithms, whereas the average processing speed for each composition ratio is 3.9 seconds for the annealing machine. Moreover, in this case, the yield of classical algorithms is 58.8%, while the yield of the annealing machine is 100%. That is, with classical algorithms, a solution is not found within the time limit of 360 minutes in some cases, whereas with the annealing machine, solutions are found for all composition ratios within the time limit of 360 minutes.

In this way, even in cases where the composition ratio is fixed, arithmetic processing using the annealing machine is faster than arithmetic processing using classical algorithms.

(Embodiment 2)

The information processing system 1000 in Embodiment 1 includes the optimization apparatus 200 as a computing apparatus. An information processing system according to the present embodiment includes a sampling apparatus as a computing apparatus. Note that components of the present embodiment which are the same as components of Embodiment 1 are denoted with the same reference signs as Embodiment 1, and detailed description thereof is reduced or omitted.

[Overall system configuration]

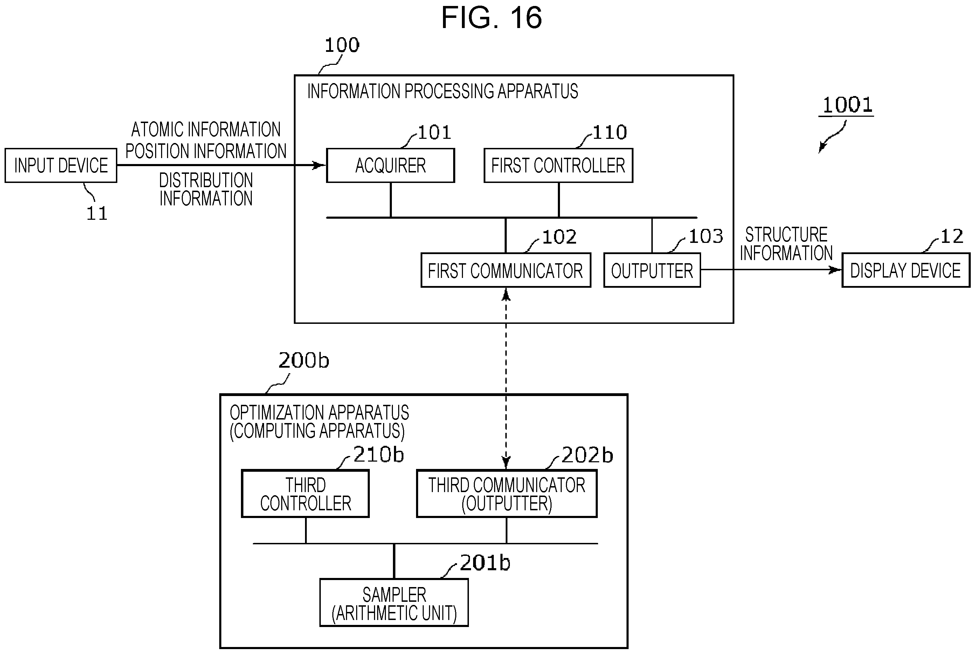

Fig. 16 is a block diagram illustrating an example of a functional configuration of the information processing apparatus 100 and a sampling apparatus included in an information processing system according to the present embodiment.

The information processing system 1001 according to the present embodiment includes the information processing apparatus 100 and a sampling apparatus 200b.

The acquirer 101 of the information processing apparatus 100 according to the present embodiment additionally acquires distribution information pertaining to a certain distribution. For example, the acquirer 101 acquires the distribution information as a signal outputted from the input device 11. One example of the certain distribution is a Boltzmann distribution, and one example of the distribution information is information indicating the temperature used in the Boltzmann distribution. Note that the certain distribution is not limited to a Boltzmann distribution. The distribution information may indicate the type of the certain distribution and parameters used to express a distribution of that type.

The first communicator 102 has similar functions as in Embodiment 1, and additionally transmits the distribution information acquired by the acquirer 101 to the sampling apparatus 200b. Also, the first communicator 102 receives combinations from the sampling apparatus 200b. Each combination includes the values of the two or more variables $\sigma$ described above (that is, $\sigma_i$ described above). That is, each combination includes the values of the variables $\sigma$ corresponding to each of the site positions indicated by the position information, and can be said to indicate the placement of each atom or the structure of atoms in an aggregate. Such combinations are combinations of the placements described above, and hereinafter may also simply be referred to as combinations. Alternatively, the combinations are also described as combinations of values of the variables $\sigma$.

The outputter 103 outputs structure information indicating structures of an aggregate that correspond respectively to the combinations. That is, for each of the combinations, the outputter 103 constructs a structure of the aggregate on the basis of the values of the two or more variables $\sigma$ included in that combination, and outputs structure information indicating the structure.

The sampling apparatus 200b is an example of a computing apparatus that receives information pertaining to the first relational expression and performs computation using the first relational expression, and executes sampling based on the certain distribution described above as the computation using the first relational expression. Specifically, the sampling apparatus 200b samples combinations in accordance with the first relational expression and the certain distribution from among all combinations that could be taken on the basis of the atomic information and the position information. In other words, the sampling apparatus 200b samples combinations in accordance with the first relational expression and the certain distribution from among all structures that an aggregate of atoms could take on the basis of the atomic information and the position information.

Such a sampling apparatus 200b includes a third controller 210b, a third communicator 202b, and a sampler 201b. The third controller 210b controls the sampler 201b and the third communicator 202b, and also executes processing other than the processing by these components.

The third communicator 202b has functions similar to the second communicator 202 of Embodiment 1, and also receives distribution information transmitted from the first communicator 102. Additionally, the third communicator 202b transmits combinations derived by the sampler 201b to the first communicator 102 of the information processing apparatus 100.

That is, the sampler 201b is an example of an arithmetic unit that derives the values of the two or more variables $\sigma$ included in the first relational expression by performing computation using the first relational expression, which represents the energy of the structure of an aggregate of atoms. Such a sampler 201b acquires the information pertaining to the first relational expression and the distribution information described above from the information processing apparatus 100 via the third communicator 202b, and performs the sampling described above with respect to the first relational expression. That is, the sampler 201b executes, with respect to the first relational expression, sampling based on the certain distribution corresponding to the distribution information, and thereby derives combinations that each include the values of the two or more variables $\sigma$.

Note that, like the optimization apparatus 200, the sampling apparatus 200b may also be configured by a processor such as a CPU, volatile memory and non-volatile memory, and a program stored in the non-volatile memory, for example. In this case, the functional configuration of the sampling apparatus 200b is realized by having the processor execute the program.

[Details of sampling]

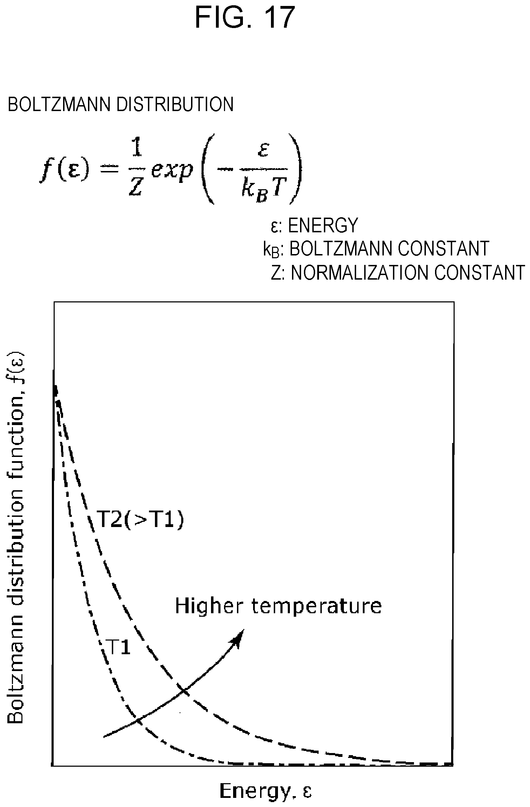

Fig. 17 is a diagram for explaining a Boltzmann distribution. Note that the graph in Fig. 17 illustrates a Boltzmann distribution, in which the vertical and horizontal axes of the graph indicate the energy $\varepsilon$ and the Boltzmann distribution function $f(\varepsilon)$, respectively. Also, the Boltzmann distribution function $f(\varepsilon)$ is indicated by Expression (3) below. In Expression (3), the energy $\varepsilon$ corresponds to the energy E in Expressions (1) and (2). Also, in Expression (3), $k_B$ is the Boltzmann constant, Z is a normalization constant, and T is a parameter indicating temperature.
[Math. 5]

$$f(\varepsilon) = \frac{1}{Z} exp\left(-\frac{\varepsilon}{k_B T}\right) \tag{3}$$

As illustrated in Fig. 17, in a Boltzmann distribution, the higher the temperature T, the greater the value of the Boltzmann distribution function $f(\varepsilon)$ at the same energy $\varepsilon$. Specifically, the value of the Boltzmann distribution function $f(\varepsilon)$ at a temperature T2 is greater than the value of the Boltzmann distribution function $f(\varepsilon)$ at a temperature T1 (where T2 > T1).

The sampler 201b of the sampling apparatus 200b samples the first relational expression according to the Boltzmann distribution corresponding to the temperature T indicated by such distribution information. That is, for each energy E, the sampler 201b samples combinations that include values of the two or more variables $\sigma$ satisfying the first relational expression (that is, Expression (1)) corresponding to that energy E, where the number of combinations to be sampled depends on the Boltzmann distribution function $f(\varepsilon=E)$ corresponding to that temperature T. As a result, the distribution of the combinations sampled by the sampler 201b is equal to the Boltzmann distribution corresponding to that temperature T.

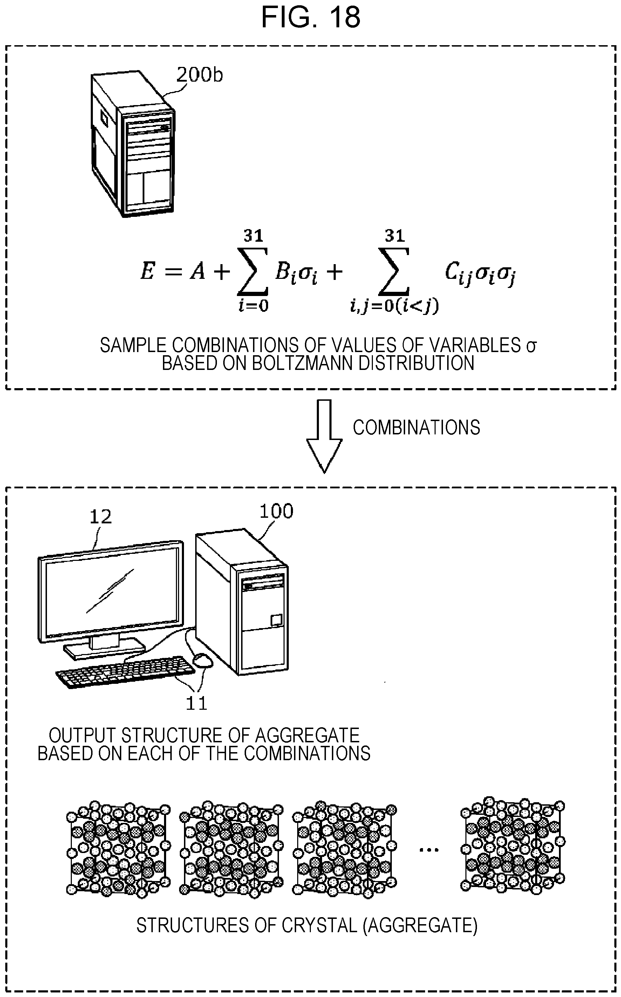

Fig. 18 is a diagram for explaining an example of processing by the sampling apparatus 200b and the information processing apparatus 100.

The sampling apparatus 200b, like the optimization apparatus 200 of Embodiment 1, receives for example the coefficients A, Bi, and $C_{ij}$ transmitted from the information processing apparatus 100. In addition, the sampling apparatus 200b receives the distribution information transmitted from the information processing apparatus 100. At this time, the sampling apparatus 200b may receive not only the coefficients, but also atomic information and position information from the information processing apparatus 100. The sampling apparatus 200b then constructs the first relational expression on the basis of the coefficients, information, and the like. Next, the sampling apparatus 200b performs, with respect to the first relational expression, sampling based on the Boltzmann distribution corresponding to the temperature indicated by the distribution information. Accordingly, the combinations described above are sampled. At this time, the sampling apparatus 200b performs the sampling described above by using an annealing machine, that is, a computer specialized in operations on Ising models, similarly to the optimization apparatus 200 of Embodiment 1. The Metropolis-Hastings algorithm may also be used in the sampling. The sampling apparatus 200b then transmits the sampled combinations to the information processing apparatus 100.

For each combination transmitted from the sampling apparatus 200b, the information processing apparatus 100 constructs a structure (crystal structure, for example) of an aggregate of atoms on the basis of that combination. That is, the information processing apparatus 100 constructs a structure of an aggregate by placing an atom of the type corresponding to the value of the variable $\sigma_i$ at the position i in the aggregate. For example, in the construction of a structure for the case of $\sigma_i=-1$ (for i=0 to 7 and 16 to 23) and $\sigma_i=+1$ (for i=8 to 15 and 24 to 31), an Au or Cu atoms is placed at each position. The information processing apparatus 100 then outputs structure information indicating the constructed structure of the aggregate for each combination. Outputting the structure information causes the structures of the aggregate to be displayed on the display device 12, for example.

[Overall process flow]

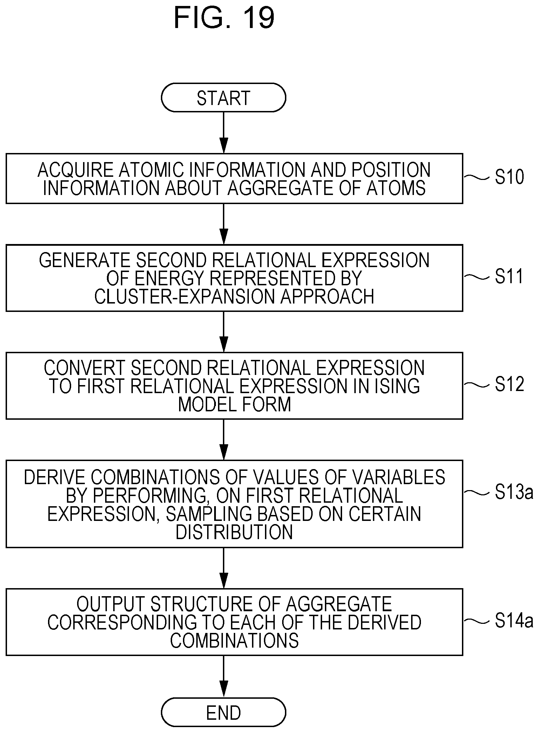

Fig. 19 is a flowchart illustrating an example of processing operations by the information processing system 1001.

(Steps S10 to S12)

The information processing apparatus 100 executes the processing in steps S10 to S12, similarly to the flowchart illustrated in Fig. 6.

(Step S13a)

The sampler 201b of the sampling apparatus 200b derives combinations by performing, with respect to the first relational expression, sampling based on a certain distribution, such as a Boltzmann distribution, for example. The combinations include values of the two or more variables σ.

(Step S14a)

For each of the derived combinations, the outputter 103 of the information processing apparatus 100 constructs a structure of the aggregate on the basis of the values of the two or more variables σ included in that combination, and outputs structure information indicating the structure. For example, the third controller 210b of the sampling apparatus 200b causes the derived combinations to be transmitted from the third communicator 202b to the information processing apparatus 100. For each of the combinations transmitted from the sampling apparatus 200b and received by the first communicator 102, the outputter 103 constructs the structure of the aggregate using that combination.

[Application of sampling]

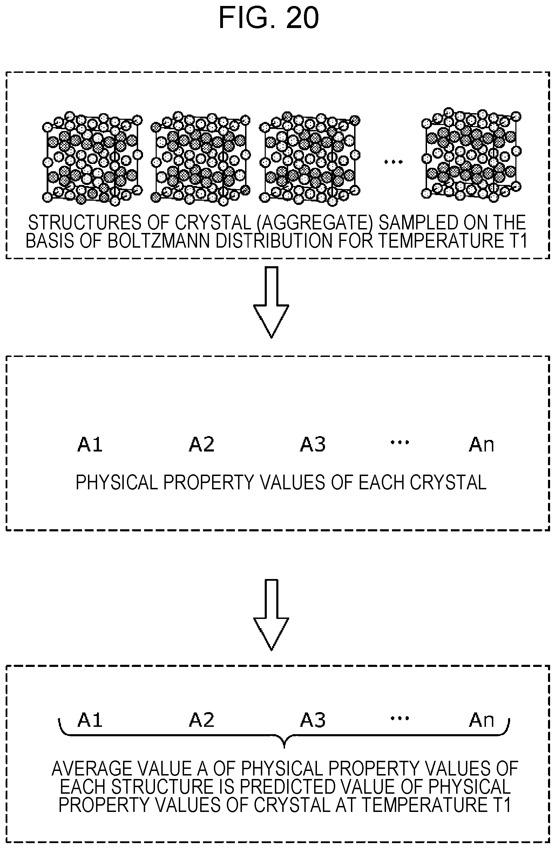

Fig. 20 is a diagram illustrating an example of additional processing operations by the first controller 110.
The first controller 110 may use the structures of an aggregate constructed by the outputter 103 to derive a predicted value of physical properties of the aggregate. For example, as illustrated in Fig. 20, structures of an aggregate are constructed on the basis of a Boltzmann distribution for the temperature T1. The first controller 110 calculates physical property values A1 to An of the structures by performing first-principles computation the like with respect to the structures. For example, the physical property values A1 to An are permittivity and the like. The first controller 110 then calculates an average value A of these physical property values A1 to An. Accordingly, a predicted value of the physical property values of the aggregate at the temperature T1 is derived.
In this way, in the present embodiment, combinations are derived by performing sampling based on a certain distribution with respect to the first relational expression including two or more variables σ that indicate the states of atoms respectively located at two or more positions in an aggregate. Note that sampling can also be referred to as searching. Also, the first relational expression may also be referred to as a function in Ising model form. Consequently, by using such a first relational expression, an annealing machine can be applied to the sampling, and the computational costs associated with the sampling can be kept low compared to the technique of the related art that uses the cluster-expansion approach and a Monte Carlo method. Also, since there is a one-to-one correspondence between the values of the two or more variables included in each of the combinations and the two or more positions in the aggregate, once the values of the two or more variables are derived, a structure of an aggregate can be constructed easily. As a result, improved efficiency can be attained in the processing for sampling or searching for the structures of aggregates of atoms.

(Embodiment 3)

An information processing system according to the present embodiment includes the optimization apparatus 200 of Embodiment 1 and the sampling apparatus 200b of Embodiment 2. Additionally, the sampling apparatus 200b according to the present embodiment uses the values of the two or more variables σ derived through energy optimization by the optimization apparatus 200 in sampling based on a certain distribution. Note that components of the present embodiment which are the same as components of Embodiment 1 or 2 are denoted with the same reference signs as Embodiment 1 or 2, and detailed description thereof is reduced or omitted.

[Overall system configuration and process flow]

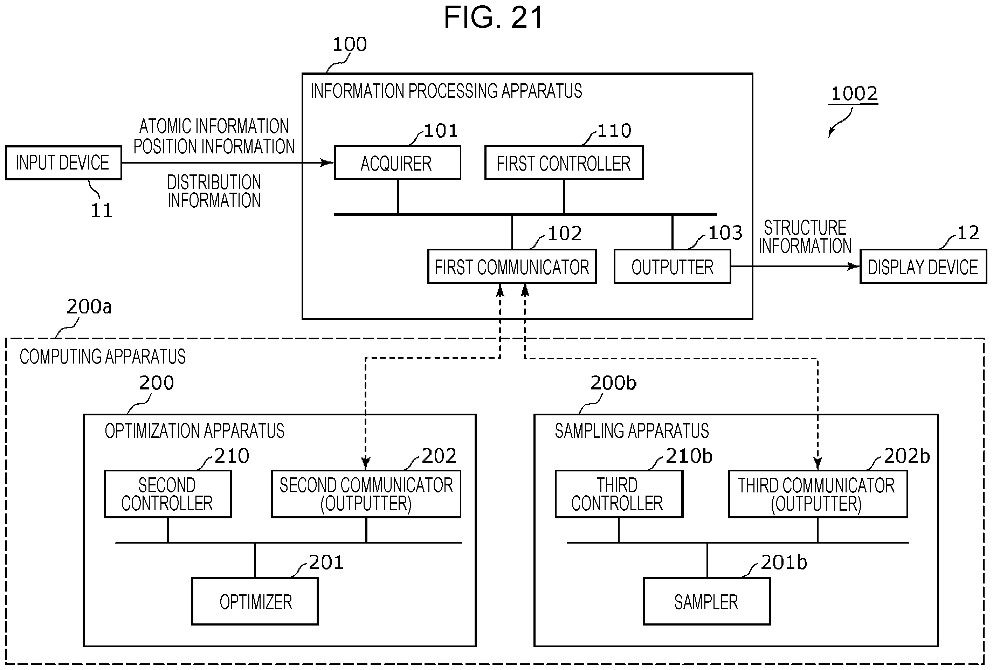

Fig. 21 is a block diagram illustrating an example of a functional configuration of the information processing apparatus 100 and a computing apparatus included in an information processing system according to the present embodiment.

The information processing system 1002 according to the present embodiment includes the information processing apparatus 100 and a computing apparatus 200a. The computing apparatus 200a includes the optimization apparatus 200 and the sampling apparatus 200b. That is, the computing apparatus 200a includes the optimization apparatus 200 that carries out a search to find an optimal solution to the first relational expression as computation using the first relational expression, and the sampling apparatus 200b that executes sampling based on a certain distribution as computation using the first relational expression.

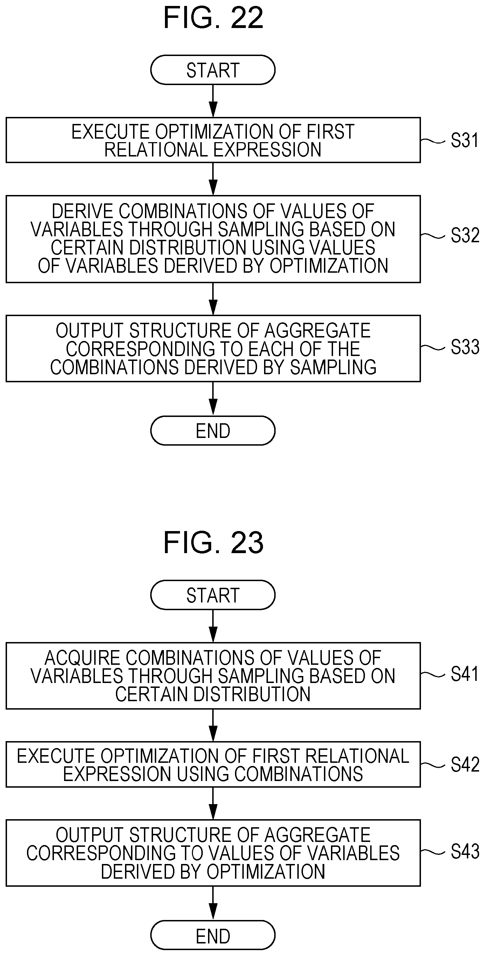

Fig. 22 is a flowchart illustrating an example of processing operations by the information processing system 1002 according to the present embodiment.

(Step S31)

First, the information processing system 1002 optimizes the energy of the first relational expression. That is, the information processing system 1002 derives the values of the two or more variables $\sigma$ with which the energy represented by the first relational expression is, for example, the lowest by performing processing similar to the processing performed by the information processing apparatus 100 and the optimization apparatus 200 in Embodiment 1. Such values of the two or more variables $\sigma$ are derived by the optimizer 201 of the optimization apparatus 200 and transmitted from the second communicator 202 to the third communicator 202b of the sampling apparatus 200b via the first communicator 102 of the information processing apparatus 100.

(Step S32)

Next, the information processing system 1002 executes, with respect to the first relational expression, sampling based on a certain distribution corresponding to the distribution information. At this time, the acquirer 101 of the information processing apparatus 100 acquires distribution information indicating the temperature T of a Boltzmann distribution, for example, and the first communicator 102 transmits the distribution information to the third communicator 202b of the sampling apparatus 200b. As a result, the third communicator 202b of the sampling apparatus 200b receives the distribution information and the values of the two or more variables $\sigma$ transmitted from the second communicator 202 of the optimization apparatus 200 in step S31. The sampler 201b of the sampling apparatus 200b uses the distribution information and the values of the two or more variables $\sigma$ received by the third communicator 202b in the sampling. Accordingly, combinations of the values of the variables $\sigma$ are derived in accordance with, for example, the Boltzmann distribution corresponding to the distribution information. The combinations derived in this way are derived by the sampler 201b of the sampling apparatus 200b and transmitted from the third communicator 202b to the first communicator 102 of the information processing apparatus 100.

(Step S33)

Next, the information processing system 1002 outputs the structure of an aggregate corresponding to each of the combinations derived in step S32. That is, for each of the combinations received by the first communicator 102, the outputter 103 of the information processing apparatus 100 constructs a structure of the aggregate on the basis of the values of the two or more variables $\sigma$ included in that combination, and outputs structure information indicating the structure.

[Sampling apparatus 200b]

The sampling apparatus 200b according to the present embodiment acquires, from the optimization apparatus 200 via the information processing apparatus 100, the values of the two or more variables $\sigma$ derived through energy optimization by the optimization apparatus 200. The sampling apparatus 200b then uses the values of the two or more variables $\sigma$ to perform sampling based on a certain distribution.

Specifically, the third communicator 202b of the sampling apparatus 200b acquires the values of the two or more variables $\sigma$ from the second communicator 202 of the optimization apparatus 200 via the first communicator 102 of the information processing apparatus 100. The sampler 201b uses the values of the two or more variables $\sigma$ acquired by the third communicator 202b to perform sampling based on a certain distribution. Accordingly, combinations are derived. That is, the sampler 201b uses the values of the two or more variables $\sigma$ derived through energy optimization as initial values of the combinations to be derived through sampling. The sampler 201b then starts sampling from the neighborhood of the initial values of the two or more variables $\sigma$.

This arrangement can raise the likelihood of realizing an improvement in the processing speed of the sampling based on a certain distribution and a reduction in the computational load, as compared to when the sampling is started from random values of the two or more variables $\sigma$.

For example, in the case of performing sampling based on a low-temperature Boltzmann distribution, if the sampling is started from random values, much computational processing may be necessary, and the processing speed may slow down. This is because if the sampling of aggregates with high-energy structures is started first despite the existence of many aggregates with low-energy structures in a low-temperature Boltzmann distribution, the computational processing for the sampling will become intensive. In other words, it will take time until the sampling of aggregates with low-energy structures is performed.

Accordingly, as described above, the information processing system 1002 according to the present embodiment optimizes the energy of the first function prior to sampling, and derives the values of the two or more variables $\sigma$ that indicate a low-energy structure (for example, states such as the types of atoms respectively located at each site position). The sampling apparatus 200b then uses the derived values of the two or more variables $\sigma$ as initial values to start sampling from the neighborhood of the initial values. With this arrangement, the sampling of aggregates with low-energy structures can be performed efficiently, making it possible to raise the likelihood of attaining an improvement in the processing speed and a reduction in the computational load of sampling.

Note that the sampler 201b of the sampling apparatus 200b may also perform sampling using the classical algorithms described above.

Similarly to Embodiment 2, the third communicator 202b outputs combinations derived through sampling by the sampler 201b to the first communicator 102 of the information processing apparatus 100.

[Information processing apparatus 100]

To cause the sampling apparatus 200b to execute the processing described above, the information processing apparatus 100 according to the present embodiment first performs processing similar to Embodiment 1. That is, as in Embodiment 1, the information processing apparatus 100 causes the optimization apparatus 200 to carry out a search to find an optimal solution to the first relational expression, or in other words to optimize the energy of the first relational expression. The information processing apparatus 100 then transmits to the sampling apparatus 200b the initial values of the two or more variables $\sigma$ derived by the energy optimization, thereby causing the sampling apparatus 200b to execute sampling based on a certain distribution using the initial values of the two or more variables $\sigma$.

In other words, the acquirer 101 of the information processing apparatus 100 according to the present embodiment additionally acquires distribution information pertaining to a certain distribution, similarly to Embodiment 2. The first communicator 102 transmits information pertaining to the first relational expression to each of the optimization apparatus 200 and the sampling apparatus 200b. In addition, the first communicator 102 receives from the optimization apparatus 200 the initial values of the two or more variables $\sigma$ derived by carrying out a search to find an optimal solution to the first relational expression. The first communicator 102 then transmits the initial values of the two or more variables $\sigma$ and the distribution information to the sampling apparatus 200b. As a result, the first communicator 102 receives combinations from the sampling apparatus 200b, each combination including values of the two or more variables $\sigma$, the combinations being derived by executing, with respect to the first relational expression, sampling based on the initial values of the two or more variables $\sigma$ and the certain distribution corresponding to the distribution information. The outputter 103 outputs structure information indicating structures of an aggregate that correspond respectively to the combinations.

With this arrangement, the values of the two or more variables $\sigma$ derived by a search to find an optimal solution to the first relational expression are used as the initial values in sampling based on a certain distribution, thereby making it possible to raise the likelihood of attaining an improvement in the processing speed and a reduction in the computational load of the sampling.

(Modification 1 of Embodiment 3)

In the example described above, the energy of the first relational expression is optimized first, after which sampling based on a certain distribution is performed. The present modification is the reverse of the example described above: sampling based on a certain distribution is performed first, after which the energy of the first relational expression is optimized. Note that the information processing system 1002 according to the present modification has the configuration illustrated in Fig. 21.

[Overall process flow]

Fig. 23 is a flowchart illustrating an example of processing operations by the information processing system 1002 according to the present modification.

(Step S41)

First, the information processing system 1002 executes, with respect to the first relational expression, sampling based on a certain distribution corresponding to the distribution information. That is, the information processing system 1002 derives combinations of the values of the two or more variables $\sigma$ by performing processing similar to the processing performed by the information processing apparatus 100 and the sampling apparatus 200b in Embodiment 2. Specifically, the acquirer 101 of the information processing apparatus 100 acquires distribution information indicating the temperature T of a Boltzmann distribution, for example, and the first communicator 102 transmits the distribution information to the third communicator 202b of the sampling apparatus 200b. The sampler 201b of the sampling apparatus 200b uses the distribution information received by the third communicator 202b in the sampling. Accordingly, combinations of the values of the variables $\sigma$ are derived in accordance with, for example, the Boltzmann distribution corresponding to the distribution information. The combinations derived in this way are derived by the sampler 201b of the sampling apparatus 200b and transmitted from the third communicator 202b to the second communicator 202 of the optimization apparatus 200 via the first communicator 102 of the information processing apparatus 100.

(Step S42)

Next, the information processing system 1002 optimizes the energy of the first relational expression. At this time, the optimizer 201 of the optimization apparatus 200 uses the combinations received by the second communicator 202 in the optimization of the energy of the first relational expression. Accordingly, the values of the two or more variables $\sigma$ with which the energy represented by the first relational expression is, for example, the lowest are derived. Such values of the two or more variables $\sigma$ are transmitted from the second communicator 202 to the first communicator 102 of the information processing apparatus 100.

(Step S43)

Next, the information processing system 1002 outputs the structure of an aggregate corresponding to the values of the two or more variables $\sigma$ derived in step S42. That is, the outputter 103 of the information processing apparatus 100 constructs a structure of the aggregate on the basis of the values of the two or more variables $\sigma$ received by the first communicator 102, and outputs structure information indicating the structure.

[Optimization apparatus 200]

The optimization apparatus 200 according to the present embodiment acquires, from the sampling apparatus 200b via the information processing apparatus 100, combinations sampled by the sampling apparatus 200b. The optimization apparatus 200 then uses the combinations to optimize the energy of the first relational expression.
Specifically, the second communicator 202 of the optimization apparatus 200 acquires combinations from the third communicator 202b of the sampling apparatus 200b via the first communicator 102 of the information processing apparatus 100. The optimizer 201 uses the combinations acquired by the second communicator 202 to optimize the energy of the first relational expression, or in other words, to carry out a search to find an optimal solution to the first relational expression. Accordingly, the values of the two or more variables $\sigma$ included in the first relational expression are derived. That is, the optimizer 201 uses the values of the two or more variables $\sigma$ included in each of the sampled combinations as the initial values of the values of the two or more variables $\sigma$ to be obtained through energy optimization. For each of the combinations, the optimizer 201 starts a search to find an optimal solution to the first relational expression, beginning from the initial values of the two or more variables $\sigma$ included in that combination.
This arrangement can raise the likelihood of attaining a reduction in the time until an optimal solution is reached, as compared to when the search (that is, the energy optimization) is started from random values of the two or more variables $\sigma$. Moreover, this arrangement can raise the likelihood of successfully finding a structure of the aggregate with lower energy as an optimal solution. For example, in the search from random values described above, there is the possibility of falling into a local solution, and it takes time to get out of the local solution. However, in the present modification, since the search is started from each of the sampled combinations, the possibility of falling into a local solution can be reduced.
The second communicator 202 outputs, to the first communicator 102 of the information processing apparatus 100, the values of the two or more variables σ derived through the search by the optimizer 201.

[Information processing apparatus 100]

To cause the optimization apparatus 200 to execute the processing described above, the information processing apparatus 100 according to the present embodiment first performs processing similar to Embodiment 2. That is, as in Embodiment 2, the information processing apparatus 100 causes the sampling apparatus 200b to execute, with respect to the first relational expression, sampling based on a certain distribution. The information processing apparatus 100 then transmits combinations derived by the sampling to the optimization apparatus 200, thereby causing the optimization apparatus 200 to execute optimization of the energy of the first relational expression using the combinations.

In other words, the acquirer 101 of the information processing apparatus 100 according to the present embodiment additionally acquires distribution information pertaining to a certain distribution, similarly to Embodiment 2. The first communicator 102 transmits information pertaining to the first relational expression to each of the optimization apparatus 200 and the sampling apparatus 200b. Furthermore, the first communicator 102 transmits distribution information to the sampling apparatus 200b. As a result, the first communicator 102 receives combinations from the sampling apparatus 200b, each combination including initial values of the two or more variables σ, the combinations being derived by executing, with respect to the first relational expression, sampling based on the certain distribution corresponding to the distribution information. The first communicator 102 transmits the combinations to the optimization apparatus 200. As a result, the first communicator 102 receives from the optimization apparatus 200 the values of the two or more variables σ derived by using the combinations to carry out a search to find an optimal solution to the first relational expression.

This arrangement can raise the likelihood of attaining a reduction in the time until an optimal solution is reached, and raise the likelihood of successfully finding a structure of the aggregate with lower energy as an optimal solution.

(Modification 2 of Embodiment 3)

Embodiment 3 and Modification 1 above may also be combined. As an example, the energy of the first relational expression is optimized, after which sampling based on a certain distribution is performed, and after that, the energy of the first relational expression is optimized again. Alternatively, sampling based on a certain distribution is performed, after which the energy of the first relational expression is optimized, and after that, sampling based on a certain distribution is performed again. That is, energy optimization and sampling are executed repeatedly in alternation. In this case, when the processing for one of either energy optimization or sampling is performed, the result of the processing for the other performed immediately previously is used. This may make it possible to execute energy optimization and sampling faster.

<Other embodiments>

The foregoing describes an information processing system according to the above embodiments and modifications, but the present disclosure is not limited to these embodiments and modifications. Embodiments such as those obtained by applying various modifications that may occur to a person skilled in the art to the above embodiments and modifications may also be included in the scope of the present disclosure insofar as such embodiments do not depart from the gist of the present disclosure. Embodiments constructed by combining components from different embodiments with each other may also be included in the present disclosure. Embodiments constructed by combining components from each of any of the embodiments and any of the modifications may also be included in the present disclosure. Furthermore, embodiments constructed by combining components from different modifications with each other may also be included in the present disclosure.

For example, in Embodiment 1 above, the information processing apparatus 100 does not perform energy optimization, and the energy optimization is performed by the optimizer 201 of the optimization apparatus 200. However, the information processing apparatus 100 may also include the optimizer 201 and perform the energy optimization. Similarly, the information processing apparatus 100 may also include the sampler 201b and perform sampling.

The above embodiments and modifications are described in terms of an example in which the aggregate, namely a crystal, includes two types of atoms, namely Au and Cu, but the types of atoms included in the aggregate are not limited thereto and may be any types. Likewise, the types of atoms included in the aggregate are not limited to two types in number, and may also be three or more types.

In the embodiments and modifications, the types of atoms are used as the states of atoms, but the states of atoms are not limited to the types of atoms. That is, the states of atoms may include at least one from among types of atoms, atomic spin states of atoms, nuclear spin states of atoms, and atomic vacancies where atoms are vacant. In this case, improved efficiency can be attained in the processing for searching for at least one from among types of atoms, atomic spin states of atoms, nuclear spin states of atoms, and atomic vacancies included in a stable aggregate of atoms. Note that there may also be three or more states of atoms. In this case, the variables take three or more values by being mapped in a one-to-one manner to the states of atoms.

In Embodiment 1 above, a single information processing apparatus 100 executes all of the processing in steps S10-S12 and S14 illustrated in Fig. 6. However, the information processing apparatus 100 may also include more than one apparatus, and each apparatus may execute the processing in any of steps S10-S12 and S14. In other words, the information processing system 1000 may include more than one information processing apparatus, and each information processing apparatus may execute a portion of the processing by the information processing apparatus 100. For example, the information processing system 1000 includes a first information processing apparatus and a second information processing apparatus which are different from one another. The first information processing apparatus acquires the first relational expression and transmits information pertaining to the first relational expression to the optimization apparatus 200. The second information processing apparatus receives the values of two or more variables derived by the optimization apparatus 200, and outputs structure information indicating the structure of an aggregate corresponding to the values of the two or more variables. Similarly, the information processing apparatus 100 may be formed from more than one information processing apparatus in each of Embodiment 2, Embodiment 3, and Modifications 1 and 2 of Embodiment 3.

Additionally, in the foregoing embodiments, each component may be configured by dedicated hardware, or realized by executing a software program suited to each component. Each component may be realized as a result of a program execution unit such as a CPU or a processor reading out and executing a software program recorded on a recording medium such as a hard disk or semiconductor memory. Herein, the program for realizing the information processing apparatus 100, the optimization apparatus 200, the sampling apparatus 200b, and the like in the above embodiments may cause a processor to execute the steps included in Figs. 6, 7, 10, 19, 22, or 23, for example.

(Hardware configuration)

Each of the information processing apparatus 100, the optimization apparatus 200, and the sampling apparatus 200b may be configured specifically by a computer system provided with a microprocessor, ROM, RAM, a hard disk drive, a display unit, a keyboard, a mouse, and the like. A program is stored in the RAM or the hard disk drive. Each of the information processing apparatus 100, the optimization apparatus 200, and the sampling apparatus 200b achieves the functions thereof as a result of the microprocessor operating in accordance with a program. The program herein is formed from a combination of instruction codes indicating commands to the computer in order to achieve a designated function.

Furthermore, some or all of the components included in each of the information processing apparatus 100, the optimization apparatus 200, and the sampling apparatus 200b may be formed from a single system large-scale integration (LSI) chip. A system LSI chip is an advanced multi-function LSI chip fabricated by integrating multiple components onto a single chip, and specifically is a computer system including a microprocessor, read-only memory (ROM), random access memory (RAM), and the like. A computer program is stored in the RAM. The system LSI chip achieves the functions thereof as a result of the microprocessor operating in accordance with the computer program.

Furthermore still, some or all of the components included in each of the information processing apparatus 100, the optimization apparatus 200, and the sampling apparatus 200b may be formed from an IC card or a separate module that can be inserted into a computer. The IC card or module is a computer system formed from a microprocessor, ROM, RAM, and the like. The IC card or module may also include the advanced multi-function LSI chip. The IC card or module achieves the functions thereof as a result of the microprocessor operating in accordance with the computer program. The IC card or the module may also be tamper-resistant.

The present disclosure may also be a method to be executed by each of the information processing apparatus 100, the optimization apparatus 200, and the sampling apparatus 200b above. The method may be realized by having a computer execute a program, or may be realized by a digital signal containing the program.

Furthermore, in the present disclosure, the program or the digital signal may also be configured as a non-transitory computer-readable recording medium. Examples of the recording medium include a flexible disk, hard disk, CD-ROM, DVD, DVD-ROM, DVD-RAM, Blu-ray® Disc (BD), and semiconductor memory. The program may also be configured as the digital signal recorded onto a non-transitory recording medium.

In addition, the present disclosure may also be configured by transmitting the program or the digital signal over an electrical communication channel, a wired or wireless communication channel, a network such as the Internet, a data broadcast, or the like.

In addition, the present disclosure may also be a computer system provided with a microprocessor and a memory, in which the memory stores a program and the microprocessor operates in accordance with the program.

Also, the program or the digital signal may be implemented by another independent computer system by being recorded onto the non-transitory recording medium and transported, or by transferring the program or the digital signal over the network or the like.

(Other 1)

"At least one from among A, B, and C" may be taken to mean "(A), (B), (C), (A and B), (A and C), (B and C), or (A and B and C)".

Industrial Applicability

The present disclosure exhibits the feature whereby improved efficiency can be attained in the processing for searching for stable structures of aggregates of atoms, and is useful in apparatuses, systems, and the like for materials development. Reference Signs List

11 input device
12 display device
100 information processing apparatus
101 acquirer
102 first communicator
103 outputter
110 first controller
200 optimization apparatus (computing apparatus)
200a computing apparatus
200b sampling apparatus (computing apparatus)
201 optimizer (arithmetic unit)
201b sampler (arithmetic unit)
202 second communicator
202b third communicator
210 second controller
210b third controller
1000, 1001, 1002 information processing system

**Claims**

1. An information processing system comprising:

   an information processing apparatus that transmits information pertaining to a relational expression representing energy of a structure of an aggregate of atoms; and
   a computing apparatus that receives the information pertaining to the relational expression and performs computation using the relational expression, wherein:

   the aggregate of atoms includes states of two or more atoms;
   the relational expression is acquired on the basis of

   (a) atomic information pertaining to the states of the two or more atoms included in the aggregate and
   (b) position information pertaining to two or more positions where any of the states of the two or more atoms in the aggregate may be respectively located,

   the relational expression including two or more variables that indicate the states of atoms respectively located at the two or more positions; and
   the information processing apparatus comprises:

   a communicator that transmits the information pertaining to the relational expression to the computing apparatus, and receives, from the computing apparatus, values of the two or more variables derived by performing computation using the relational expression; and
   an outputter that outputs structure information indicating a structure of the aggregate that corresponds to the values of the two or more variables.

2. An information processing apparatus comprising:

an acquirer that acquires a first relational expression including two or more variables that indicate states of atoms respectively located at two or more positions included in an aggregate of atoms as a relational expression representing energy of a structure of the aggregate, the first relational expression being acquired on the basis of

(a) atomic information pertaining to the states of the two or more atoms included in the aggregate and
(b) position information pertaining to two or more positions where any of the states of the two or more atoms in the aggregate may be respectively located;

a communicator that transmits the information pertaining to the first relational expression to a computing apparatus that performs computation using the first relational expression, and receives, from the computing apparatus, values of the two or more variables derived by performing computation using the first relational expression; and

an outputter that outputs structure information indicating a structure of the aggregate that corresponds to the values of the two or more variables.

3. The information processing apparatus according to claim 2, wherein the computing apparatus is an optimization apparatus that carries out a search to find an optimal solution to the first relational expression as the computation using the first relational expression.

4. The information processing apparatus according to claim 2, wherein the states of the atoms include at least one from among types of the atoms, spin states of the atoms, and atomic vacancies where the atoms are vacant.

5. The information processing apparatus according to claim 2, wherein the first relational expression is represented by [Math. 1]

$$E = A + \sum_i B_i \sigma_i + \sum_{i,j} C_{ij} \sigma_i \sigma_j \qquad (1)$$

where

E indicates energy of a structure of the aggregate,
i and j each indicate the position in the aggregate,
$\sigma_i$ is the variable at position i in the aggregate,
$\sigma_j$ is the variable at position j in the aggregate,
A is a zeroth-order cluster interaction coefficient,
$B_i$ is a first-order cluster interaction coefficient, and
$C_{ij}$ is a second-order cluster interaction coefficient.

6. The information processing apparatus according to claim 2, wherein:

the first relational expression represents the energy by the sum of a first coefficient and n (where n is an integer equal to or greater than 2) terms; and
among the n terms, the kth (where k is an integer equal to or greater than 1 and less than or equal to n) term is the sum of the products respectively corresponding to groups of numbers that could be taken from the two or more variables, each of the groups containing k variables, and each of the products being the product of the k variables included in a group multiplied by a second coefficient corresponding to that group.

7. The information processing apparatus according to claim 2, wherein the acquirer acquires the first relational expression by converting a second relational expression to an Ising model, the second relational expression being represented using the cluster-expansion approach on the basis of the atomic information and the position information.

8. The information processing apparatus according to claim 7, wherein the second relational expression is represented by
[Math. 2]

$$E = \sum_{\alpha} V_{\alpha} \varphi_{\alpha}$$

$$(2)$$

where

E indicates energy of a structure of the aggregate,
$\alpha$ is a cluster identifier,
$V_{\alpha}$ is a coefficient indicating the magnitude of the contribution of cluster $\alpha$ to the energy, and
$\phi_{\alpha}$ is a correlation function of cluster $\alpha$.

9. The information processing apparatus according to claim 2, wherein:

the computing apparatus is a sampling apparatus that executes sampling based on a certain distribution as the computation using the first relational expression;
the acquirer further acquires distribution information pertaining to the certain distribution;
the communicator further

transmits the distribution information to the sampling apparatus, and
in the receiving of the values of the two or more variables, receives combinations from the sampling apparatus, each combination including values of the two or more variables, the combinations being derived by executing, with respect to the first relational expression, sampling based on the certain distribution corresponding to the distribution information; and

the outputter outputs structure information indicating structures of the aggregate that correspond respectively to the combinations.

10. The information processing apparatus according to claim 2, wherein:

the computing apparatus comprises:

an optimization apparatus that carries out a search to find an optimal solution to the first relational expression as the computation using the first relational expression and
a sampling apparatus that executes sampling based on a certain distribution as the computation using the first relational expression;

the acquirer further acquires distribution information pertaining to the certain distribution;
the communicator,

in the transmitting of the information pertaining to the first relational expression,

transmits the information pertaining to the first relational expression to each of the optimization apparatus and the sampling apparatus,
further receives, from the optimization apparatus, initial values of the two or more variables derived by carrying out a search to find an optimal solution to the first relational expression, and
transmits the initial values of the two or more variables and the distribution information to the sampling apparatus; and

in the receiving of the values of the two or more variables, receives combinations from the sampling apparatus, each combination including values of the two or more variables, the combinations being derived by executing, with respect to the first relational expression, sampling based on the initial values of the two or more variables and the certain distribution corresponding to the distribution information; and

the outputter outputs structure information indicating structures of the aggregate that correspond respectively to the combinations.

11. The information processing apparatus according to claim 2, wherein:

the computing apparatus comprises:

an optimization apparatus that carries out a search to find an optimal solution to the first relational expression as the computation using the first relational expression and
a sampling apparatus that executes sampling based on a certain distribution as the computation using the first relational expression;

the acquirer further acquires distribution information pertaining to the certain distribution;
the communicator,

in the transmitting of the information pertaining to the first relational expression,

transmits the information pertaining to the first relational expression to each of the optimization apparatus and the sampling apparatus,
further transmits the distribution information to the sampling apparatus,
receives combinations from the sampling apparatus, each combination including initial values of the two or more variables, the combinations being derived by executing, with respect to the first relational expression, sampling based on the certain distribution corresponding to the distribution information, and transmits the combinations to the optimization apparatus, and

in the receiving of the values of the two or more variables, receives, from the optimization apparatus, values of the two or more variables derived by using the combinations to carry out a search to find an optimal solution to the first relational expression.

12. A computing apparatus comprising:

an arithmetic unit that derives values of two or more variables included in a relational expression by performing computation using the relational expression, the relational expression representing energy of a structure of an aggregate of atoms; and
an outputter that outputs the values of the two or more variables derived by the arithmetic unit, wherein:
the relational expression is acquired on the basis of

(a) atomic information pertaining to the states of two or more atoms included in the aggregate and
(b) position information pertaining to two or more positions where any of the states of the two or more atoms in the aggregate may be respectively located,

the relational expression including the two or more variables that indicate the states of atoms respectively located at the two or more positions.

13. The computing apparatus according to claim 12, wherein the arithmetic unit carries out a search to find an optimal solution to the relational expression as the computation using the relational expression.

14. The computing apparatus according to claim 12, wherein:

the arithmetic unit derives combinations by executing, with respect to the relational expression, sampling based on a certain distribution as the computation using the relational expression;
the outputter outputs the combinations; and
each of the combinations includes values of the two or more variables included in the relational expression.

15. An information processing method to be carried out by a computer, the method comprising:

acquiring a relational expression including two or more variables that indicate states of atoms respectively located at two or more positions included in an aggregate of atoms as a relational expression representing energy of a structure of the aggregate, the relational expression being acquired on the basis of

(a) atomic information pertaining to the states of the two or more atoms included in the aggregate and
(b) position information pertaining to two or more positions where any of the states of the two or more atoms in the aggregate may be respectively located;

transmitting information pertaining to the relational expression to a computing apparatus that performs computation using the relational expression;

receiving, from the computing apparatus, values of the two or more variables derived by performing computation using the relational expression; and

outputting structure information indicating a structure of the aggregate that corresponds to the values of the two or more variables.

16. An information processing method to be carried out by a computer, the method comprising:

acquiring a relational expression including two or more variables that indicate states of atoms respectively located at two or more positions included in an aggregate of atoms as a relational expression representing energy of a structure of the aggregate, the relational expression being acquired on the basis of

(a) atomic information pertaining to the states of the two or more atoms included in the aggregate and
(b) position information pertaining to two or more positions where any of the states of the two or more atoms in the aggregate may be respectively located; and transmitting information pertaining to the relational expression to a computing apparatus that performs computation using the relational expression, wherein:

the computing apparatus is the computing apparatus according to claim 12.

17. An information processing method to be carried out by a computer, the method comprising:

receiving values of the two or more variables derived by the computing apparatus according to claim 12; and
outputting structure information indicating a structure of the aggregate that corresponds to the values of the two or more variables.

18. A computing method to be carried out by a computer, the method comprising:

deriving values of two or more variables included in a relational expression by performing computation using the relational expression, the relational expression representing energy of a structure of an aggregate of atoms; and
outputting the derived values of the two or more variables, wherein:

the relational expression is acquired on the basis of

(a) atomic information pertaining to the states of two or more atoms included in the aggregate and
(b) position information pertaining to two or more positions where any of the states of the two or more atoms in the aggregate may be respectively located,

the relational expression including the two or more variables that indicate the states of atoms respectively located at the two or more positions.

19. The computing method according to claim 18, wherein, in the deriving, a search is carried out to find an optimal solution to the relational expression as the computation using the relational expression.

20. The computing method according to claim 18, wherein:

in the deriving, combinations are derived by executing, with respect to the relational expression, sampling based on a certain distribution as the computation using the relational expression;
in the outputting, the combinations are outputted; and
each of the combinations includes values of the two or more variables included in the relational expression.

21. The computing method according to claim 18, wherein:

in the deriving, initial values of the two or more variables included in the relational expression are derived by a carrying out a search to find an optimal solution to the relational expression as the computation using the relational expression, and combinations are derived by executing, with respect to the relational expression, sampling based on a certain distribution as computation using the derived initial values of the two or more variables;
in the outputting, the combinations are outputted; and

each of the combinations includes values of the two or more variables included in the relational expression.

22. The computing method according to claim 18, wherein, in the deriving, combinations are derived by executing, with respect to the relational expression, sampling based on a certain distribution as the computation using the relational expression, each combination including initial values of the two or more variables, and values of the two or more variables included in the relational expression are derived by a carrying out a search to find an optimal solution to the first relational expression as computation using the derived combinations.

# FIG. 1

## FIG. 2

INFORMATION PROCESSING APPARATUS — 100

ATOMIC INFORMATION
POSITION INFORMATION

INPUT DEVICE — 11

ACQUIRER — 101

FIRST CONTROLLER — 110

FIRST COMMUNICATOR — 102

OUTPUTTER — 103

STRUCTURE INFORMATION

DISPLAY DEVICE — 12

OPTIMIZATION APPARATUS
(COMPUTING APPARATUS) — 200

SECOND CONTROLLER — 210

SECOND COMMUNICATOR
(OUTPUTTER) — 202

OPTIMIZER
(ARITHMETIC UNIT) — 201

EP 4 495 937 A1

# FIG. 3

(a)

(b)

$$E = \sum_{\alpha} V_{\alpha} \cdot \varphi_{\alpha}$$

SECOND RELATIONAL EXPRESSION REPRESENTED
USING CLUSTER-EXPANSION APPROACH

EXTRACT COEFFICIENTS A, B, C

(c)

$$E = A + \sum_{i=0}^{31} B_i \sigma_i + \sum_{i,j=0(i<j)}^{31} C_{ij} \sigma_i \sigma_j$$

FIRST RELATIONAL EXPRESSION
IN ISING MODEL FORM

Au   Cu

$\sigma_i = +1$ (Au)
$\sigma_i = -1$ (Cu)

CRYSTAL (AGGREGATE)

# FIG. 4

| COEFFICIENT A |
|---|
| −0.0173730000 |

| COEFFICIENT B | i |
|---|---|
| 0.0000624375 | 0 |
| 0.0000624375 | 1 |
| 0.0000624375 | 2 |
| 0.0000624375 | 3 |
| 0.0000624375 | 4 |
| 0.0000624375 | 5 |
| 0.0000624375 | 6 |
| 0.0000624375 | 7 |
| 0.0000624375 | 8 |
| 0.0000624375 | 9 |
| 0.0000624375 | 10 |
| 0.0000624375 | 11 |
| 0.0000624375 | 12 |
| 0.0000624375 | 13 |
| 0.0000624375 | 14 |
| 0.0000624375 | 15 |
| 0.0000624375 | 16 |
| 0.0000624375 | 17 |
| 0.0000624375 | 18 |
| 0.0000624375 | 19 |
| 0.0000624375 | 20 |
| 0.0000624375 | 21 |
| 0.0000624375 | 22 |
| 0.0000624375 | 23 |
| 0.0000624375 | 24 |
| 0.0000624375 | 25 |
| 0.0000624375 | 26 |
| 0.0000624375 | 27 |
| 0.0000624375 | 28 |
| 0.0000624375 | 29 |
| 0.0000624375 | 30 |
| 0.0000624375 | 31 |

| COEFFICIENT C | i | j |
|---|---|---|
| −0.0002364375 | 0 | 0 |
| 0.0000598125 | 0 | 1 |
| 0.0001606875 | 0 | 2 |
| 0.0000598125 | 0 | 3 |
| 0.0000598125 | 0 | 4 |
| 0.0002891250 | 0 | 5 |
| −0.0003870000 | 0 | 6 |
| 0.0000598125 | 0 | 7 |
| −0.0003675625 | 0 | 8 |
| 0.0002891250 | 0 | 9 |
| 0.0000598125 | 0 | 10 |
| 0.0000598125 | 0 | 11 |
| 0.0000598125 | 0 | 12 |
| 0.0001606875 | 0 | 13 |

# FIG. 5

$$E = A + \sum_{i=0}^{31} B_i \sigma_i + \sum_{i,j=0(i<j)}^{31} C_{ij} \sigma_i \sigma_j$$

DETERMINE VALUES OF VARIABLES σ FOR
WHICH ENERGY IS MINIMIZED

VALUES OF VARIABLES σ

OUTPUT STRUCTURE OF AGGREGATE
BASED ON VALUES OF VARIABLES σ

Au

Cu

CRYSTAL (AGGREGATE)

# FIG. 6

```
          ┌─────────────┐
          │    START    │
          └─────────────┘
                 │
                 ▼
┌───────────────────────────────────────┐
│  ACQUIRE ATOMIC INFORMATION AND POSITION │──── S10
│  INFORMATION ABOUT AGGREGATE OF ATOMS    │
└───────────────────────────────────────┘
                 │
                 ▼
┌───────────────────────────────────────┐
│  GENERATE SECOND RELATIONAL EXPRESSION  │──── S11
│       OF ENERGY REPRESENTED BY          │
│      CLUSTER-EXPANSION APPROACH         │
└───────────────────────────────────────┘
                 │
                 ▼
┌───────────────────────────────────────┐
│  CONVERT SECOND RELATIONAL EXPRESSION   │──── S12
│  TO FIRST RELATIONAL EXPRESSION IN ISING │
│             MODEL FORM                   │
└───────────────────────────────────────┘
                 │
                 ▼
┌───────────────────────────────────────┐
│  USE ANNEALING MACHINE TO DERIVE VALUES OF │──── S13
│  VARIABLES THAT MINIMIZE ENERGY OF FIRST   │
│         RELATIONAL EXPRESSION              │
└───────────────────────────────────────┘
                 │
                 ▼
┌───────────────────────────────────────┐
│     OUTPUT STRUCTURE OF AGGREGATE       │──── S14
│   CORRESPONDING TO DERIVED VALUES       │
│            OF VARIABLES                  │
└───────────────────────────────────────┘
                 │
                 ▼
          ┌─────────────┐
          │     END     │
          └─────────────┘
```

# FIG. 7

S11

```
                    ┌─────────────┐
                    │    START    │
                    └─────────────┘
                           │
                           ▼
S111  ┌──────────────────────────────────────────┐
      │ ACQUIRE SYMMETRICALLY INDEPENDENT         │
      │ CLUSTERS (FIGURES) FROM                   │
      │ POSITION INFORMATION                      │
      └──────────────────────────────────────────┘
                           │
                           ▼
S112  ┌──────────────────────────────────────────┐
      │ DETERMINE CLUSTERS $\alpha_{max}$ OF      │
      │ MAXIMUM SIZE TO BE USED                   │
      └──────────────────────────────────────────┘
                           │
                           ▼
S113  ┌──────────────────────────────────────────┐
      │ GENERATE N STRUCTURES OF SMALLER          │
      │ SIZE THAN COMPUTATION TARGET              │
      └──────────────────────────────────────────┘
                           │
                           ▼
S114  ┌──────────────────────────────────────────┐
      │ FOR EACH OF N STRUCTURES, COMPUTE         │
      │ ENERGY E AND CORRELATION $\varphi_\alpha$ FOR │
      │ CLUSTERS INCLUDED IN CLUSTERS $\alpha_{max}$ │
      │ OF MAXIMUM SIZE                           │
      └──────────────────────────────────────────┘
                           │
                           ▼
S115  ┌──────────────────────────────────────────┐
      │ FROM N PAIRS OF ($\varphi_\alpha$, E) DATA, OBTAIN │
      │ COEFFICIENT $V_\alpha$ OF SECOND RELATIONAL │
      │ EXPRESSION E = $\Sigma V_\alpha \varphi_\alpha$ USING METHOD OF │
      │ LEAST SQUARES                             │
      └──────────────────────────────────────────┘
                           │
                           ▼
S116        ◇ PRECISION SUFFICIENT? ◇ ──── No
                           │
                          Yes
                           ▼
                    ┌─────────────┐
                    │     END     │
                    └─────────────┘
```

# FIG. 8

# FIG. 9

EP 4 495 937 A1

[PROCESSING IN STEP S114]

$E(\ \odot\ )$     $E(\ \bullet\ )$     $E(\ \bullet\!-\!\bullet\ )$     $E(\ \bullet\!-\!\odot\ )$

$\varphi_0 = 1$

$\varphi_1(\ \odot\ ) = 1/4$        $\varphi_1(\ \bullet\ ) = -1/4$

$\varphi_2(\ \bullet\!-\!\bullet\ ) = 1/4$        $\varphi_2(\ \bullet\!-\!\odot\ ) = -1/4$

[PROCESSING IN STEP S115]

$$E = V_0 \varphi_0 + V_1 \varphi_1 + V_2 \varphi_2 \qquad (\varphi_0 = 1)$$

# FIG. 10

S12

START

S121 — ACQUIRE CLUSTERS ($\alpha_{max}$ AND SUB-CLUSTERS INCLUDED THEREIN) WITH CONSIDERATION FOR WHEN SECOND RELATIONAL EXPRESSION IS GENERATED

S122 — SEARCH FOR ONE SUCH CLUSTER $\alpha$ AMONG SITES OF STRUCTURE TO BE COMPUTED, ACQUIRE ALL SITES (i, j, ..., n) WHERE POINTS FORMING CLUSTER ARE LOCATED

S123 — REPRESENT COEFFICIENT $\sigma_i\sigma_j...\sigma_n$ OF ISING MODEL USING $V_\alpha$

S124 — PERFORM SIMILAR OPERATIONS FOR ALL $\alpha$

END

FIG. 11

[PROCESSING IN STEP S121]
  EXAMPLE OF FIRST-ORDER AND
  SECOND-ORDER CLUSTERS

FIG. 12

[PROCESSING IN STEP S122]
  DISPLAY SEVERAL RESULTS OF SEARCH
  FOR α=2 CLUSTERS

[PROCESSING IN STEP S123]

$c_2 V_2 (\sigma_0 \sigma_2 + \sigma_1 \sigma_3 + \sigma_4 \sigma_6 + ...)$ IS INCLUDED IN
  TERMS IN ISING FUNCTION

FIG. 13

[PROCESSING IN STEP S122]
DISPLAY SEVERAL RESULTS OF SEARCH
FOR α=3 CLUSTERS

[PROCESSING IN STEP S123]
$c_3 V_3(\sigma_0\sigma_1 + \sigma_2\sigma_3 + \sigma_4\sigma_5 + ...)$ IS INCLUDED IN
TERMS IN ISING FUNCTION

FIG. 14

[PROCESSING IN STEP S121]
EXAMPLE OF THIRD-ORDER CLUSTER

$\alpha = M$

# FIG. 15

[PROCESSING IN STEP S122]
  DISPLAY SEVERAL RESULTS OF
  SEARCH FOR α=M CLUSTERS

[PROCESSING IN STEP S123]
  $c_M V_M (\sigma_0 \sigma_1 \sigma_2 + \sigma_4 \sigma_5 \sigma_6 + \sigma_0 \sigma_{16} \sigma_{26} + ...)$ IS
  INCLUDED IN TERMS IN ISING FUNCTION

## FIG. 16

EP 4 495 937 A1

# FIG. 17

BOLTZMANN DISTRIBUTION

$$f(\varepsilon) = \frac{1}{Z} exp\left(-\frac{\varepsilon}{k_B T}\right)$$

$\varepsilon$: ENERGY
$k_B$: BOLTZMANN CONSTANT
Z: NORMALIZATION CONSTANT

Boltzmann distribution function, $f(\varepsilon)$ — vertical axis

T2(>T1)

Higher temperature

T1

Energy, $\varepsilon$ — horizontal axis

# FIG. 18

200b

$$E = A + \sum_{i=0}^{31} B_i \sigma_i + \sum_{i,j=0(i<j)}^{31} C_{ij} \sigma_i \sigma_j$$

SAMPLE COMBINATIONS OF VALUES OF VARIABLES σ
BASED ON BOLTZMANN DISTRIBUTION

COMBINATIONS

12    100

11

OUTPUT STRUCTURE OF AGGREGATE
BASED ON EACH OF THE COMBINATIONS

...

STRUCTURES OF CRYSTAL (AGGREGATE)

# FIG. 19

```
                    ┌─────────────┐
                    │    START    │
                    └─────────────┘
                           │
                           ▼
    ┌──────────────────────────────────────────┐
    │  ACQUIRE ATOMIC INFORMATION AND POSITION  │      S10
    │  INFORMATION ABOUT AGGREGATE OF ATOMS     │
    └──────────────────────────────────────────┘
                           │
                           ▼
    ┌──────────────────────────────────────────┐
    │  GENERATE SECOND RELATIONAL EXPRESSION    │
    │       OF ENERGY REPRESENTED BY            │      S11
    │       CLUSTER-EXPANSION APPROACH          │
    └──────────────────────────────────────────┘
                           │
                           ▼
    ┌──────────────────────────────────────────┐
    │  CONVERT SECOND RELATIONAL EXPRESSION     │
    │  TO FIRST RELATIONAL EXPRESSION IN ISING  │      S12
    │              MODEL FORM                   │
    └──────────────────────────────────────────┘
                           │
                           ▼
    ┌──────────────────────────────────────────┐
    │  DERIVE COMBINATIONS OF VALUES OF VARIABLES│
    │  BY PERFORMING, ON FIRST RELATIONAL       │      S13a
    │  EXPRESSION, SAMPLING BASED ON CERTAIN    │
    │              DISTRIBUTION                  │
    └──────────────────────────────────────────┘
                           │
                           ▼
    ┌──────────────────────────────────────────┐
    │      OUTPUT STRUCTURE OF AGGREGATE        │
    │  CORRESPONDING TO EACH OF THE DERIVED     │      S14a
    │              COMBINATIONS                 │
    └──────────────────────────────────────────┘
                           │
                           ▼
                    ┌─────────────┐
                    │     END     │
                    └─────────────┘
```

# FIG. 20

STRUCTURES OF CRYSTAL (AGGREGATE) SAMPLED ON THE
BASIS OF BOLTZMANN DISTRIBUTION FOR TEMPERATURE T1

A1        A2        A3        ...        An

PHYSICAL PROPERTY VALUES OF EACH CRYSTAL

A1        A2        A3        ...        An

AVERAGE VALUE A OF PHYSICAL PROPERTY VALUES OF
EACH STRUCTURE IS PREDICTED VALUE OF PHYSICAL
PROPERTY VALUES OF CRYSTAL AT TEMPERATURE T1

# FIG. 21

# FIG. 22

START

EXECUTE OPTIMIZATION OF FIRST RELATIONAL EXPRESSION — S31

DERIVE COMBINATIONS OF VALUES OF VARIABLES THROUGH SAMPLING BASED ON CERTAIN DISTRIBUTION USING VALUES OF VARIABLES DERIVED BY OPTIMIZATION — S32

OUTPUT STRUCTURE OF AGGREGATE CORRESPONDING TO EACH OF THE COMBINATIONS DERIVED BY SAMPLING — S33

END

# FIG. 23

START

ACQUIRE COMBINATIONS OF VALUES OF VARIABLES THROUGH SAMPLING BASED ON CERTAIN DISTRIBUTION — S41

EXECUTE OPTIMIZATION OF FIRST RELATIONAL EXPRESSION USING COMBINATIONS — S42

OUTPUT STRUCTURE OF AGGREGATE CORRESPONDING TO VALUES OF VARIABLES DERIVED BY OPTIMIZATION — S43

END

## INTERNATIONAL SEARCH REPORT

| | International application No. |
| --- | --- |
| | **PCT/JP2023/006506** |

### A. CLASSIFICATION OF SUBJECT MATTER

*G16C 20/40*(2019.01)i
FI:  G16C20/40

According to International Patent Classification (IPC) or to both national classification and IPC

### B. FIELDS SEARCHED

Minimum documentation searched (classification system followed by classification symbols)

G16C10/00 - 99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2021-032617 A (YAMAGATA UNIVERSITY) 01 March 2021 (2021-03-01) paragraphs [0016]-[0019], [0026]-[0050] | 1-4, 12-13, 15-19 |
| A | entire text, all drawings | 5-11, 14, 20-22 |
| A | ARTEM, Oganov R. et al. Crystal structure prediction using evolutionary algorithms: principles and applications, [online], 2006, pp. 1-40, [retrieved on: 4 April 2023], <URL:https://arxiv.org/ftp/arxiv/papers/0911/0911.3186.pdf> entire text, all drawings | 1-22 |

☐ Further documents are listed in the continuation of Box C.  ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **04 April 2023** | **18 April 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

| INTERNATIONAL SEARCH REPORT | | International application No. | |
|---|---|---|---|
| Information on patent family members | | **PCT/JP2023/006506** | |

| Patent document cited in search report | Publication date (day/month/year) | Patent family member(s) | Publication date (day/month/year) |
|---|---|---|---|
| JP 2021-032617 A | 01 March 2021 | (Family: none) | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021127418 A
- JP 2021028780 A

- JP 2021033768 A

**Non-patent literature cited in the description**

- **ZIJIAN YANG** ; **ROBYN E. WARD** ; **NAOTO TANIBATA** ; **HAYAMI TAKEDA** ; **MASANOBU NAKAYAMA** ; **TORU ASAKA**. Arrangement in La1/3NbO3 Obtained by First-Principles Density Functional Theory with Cluster Expansion and Monte Carlo Simulation. *Journal of Physical Chemistry C*, 2020, vol. 124, 9746

- High-Entropy Alloys-Various New Properties Generated by the Cocktail Effect. Uchida Rokakuho, 01 May 2020